# EUROPEAN PATENT APPLICATION

(11) **EP 2 389 941 A1**
(43) Date of publication of application: **30.11.2011**
(21) Application number: 11166687.1
(22) Date of filing: 18.08.2005
(51) Int. Cl.: A61K 38/00, A61K 38/02, A61K 38/05

(54) **Growth-hormone secretagogues**

(30) Priority: 18.08.2004 US 602713 P; 22.10.2004 US 621343 P
(62) Divisional of application: 05786392.0
(71) Applicant: Elixir Pharmaceuticals, Inc., Cambridge, MA 02139 (US)
(72) Inventor: Geesaman, Bard J., Cambridge, MA 02139 (US)
(74) Representative: Williams, Gareth Owen

(57) **Abstract**

Disclosed are methods of treating a disorder of the stomach, intestine or duodenum in a human subject. The method includes administering to the subject, a pharmaceutical composition comprising a compound that increases gastrointestinal motility (e.g., by modulating myenteric nerve activity) and/or induces the secretion or production of growth hormone.

## Description

This application claims priority to U.S. Application Serial No. 60/602,713, filed August 18, 2004 and Serial No. 60/621,343, filed October 22, 2004, the contents of both of which are hereby incorporated by reference in their entireties.

Ghrelin has many physiological effects including increasing appetite, stimulating growth hormone release, and increasing gastric motility. Thus, ghrelin receptor agonists are useful in various clinical applications including cachexia, ileus, gastroparesis, and HIV lipodystrophy.

In humans, growth hormone (GH) is essential for linear growth of the infant, child, and adolescent and also plays an important role in the regulation of metabolism. Growth hormone is a 22 kDa, 191 amino acid single chain peptide containing two disulfide bridges, produced from a larger precursor. GH secretion can be increased, e.g., by stimulating or inhibiting various neurotransmitter systems in the brain and hypothalamus.

Methods for treating and preventing stomach and intestinal disorders and dysfunctions, cachexia and lipodystrophy using a compound, e.g., a compound that induces the production or secretion of GH and/or the modulation of mesenteric nerve activity, e.g., myenteric nerve activity, are described herein. In one aspect, the method includes administering, to a subject, having or at risk for a stomach or intestinal disorder a compound that modulates mesenteric nerve activity, e.g., myenteric nerve activity, in the subject, e.g., a compound described herein.. The compound can be administered in an amount effective to treat or prevent the disorder. In another aspect, the method includes administering, to a subject, having or at risk for cachexia or lipodystrophy a compound that induces the production or secretion of GH and IGF-1 in the subject, e.g., a compound described herein. The compound can be administered in an amount effective to treat or prevent the disorder. For example, the compound can be administered as a pharmaceutical composition.

In one aspect, the invention includes a method of treating or preventing a disorder of the stomach, intestine (e.g., small intestine or large intestine) or duodenum, or generally a disorder in which transit through the digestive system (e.g., the stomach or small intestine) is compromised. The disorder can be caused, for example, by damage to a nerve that contributes to contraction of the stomach or small intestine, such as the vagus nerve. Moreover, the disorder can be chronic or acute. Treatment of the disorder is not limited by the cause thereof. In some embodiments, dysfunction occurs in a post-operative patient, e.g., where surgical intervention has resulted in gastric or colonic motility disturbances. In other embodiments, the dysfunction is associated with intraperitoneal or retroperitoneal infection, mesenteric ischemia, by arterial or venous injury, retroperitoneal or intra-abdominal hematomas, intra-abdominal surgery, renal or thoracic disease, or metabolic disturbances (e.g., hypokalemia). In some embodiments, the dysfunction occurs as a result of a chronic condition, such as diabetes, which can result in nerve damage to the stomach or intestine. Representative examples of disorders include ileus (e.g., opioid-induced ileus and/or post-operative ileus) and gastroparesis (e.g., surgically-induced gastroparesis, infectious gastroparesis, drug-induced gastroparesis, idiopathic gastroparesis, central nervous system gastroparesis, and metabolic gastroparesis, e.g., diabetic gastroparesis including that occurring in subjects having either type 1 or type 2 diabetes).

In one aspect, the invention includes a method of treating or preventing cachexia, for example, cancer cachexia. The cachexia can result, for example, from a chronic illness such as cancer, AIDS, or anorexia, or from a treatment regime, such as a cancer or AIDS treatment regime.

In one aspect, the invention includes a method of treating or preventing lipodystrophy (e.g., HIV lipodystrophy). The lipodystrophy can be acquired, for example, lipodystrophy associated with HIV therapy, or the lipodystrophy can be familial or genetic. Treatment of the lipodystrophy is not limited by the cause thereof. In some embodiments, the lipodystrophy is associated with HIV protease inhibitor (PI) therapy and/or nucleoside inhibitor therapy. Other representative examples of lipodystrophy include Congenital Generalized Lipodystrophy (CGL), Familial Partial Lipodystrophy Dunnigan variety (FPLD), FPL Mandibuloacral Dysplasia, Kobberling, Multiple Symmetric Lipomatosis (MSL, Madelung's disease), SHORT Syndrome, and Neonatal Progeroid Syndrome (Wiedemann-Rautenstrauch Syndrome).

Compounds that agonize (e.g., activate) the ghrelin receptor (which, for example, stimulates the production/secretion of GH), compounds that agonize the GH receptor, compounds that bind to the growth hormone releasing hormone (GHRH) receptor, compounds that agonize the GHRH receptor, compounds that function as somatostatin antagonists, and compounds that function as GH secretagogue agonists. Examples of such compounds include those compounds having the structure described in Table 1 (e.g., compound 1, compound 2, compound 3, compound 4, compound 5, compound 6, compound 7, compound 8, compound 9, compound 10, compound 11, and compound 12). These compounds include corresponding salts, prodrugs (including prodrug esters), stereoisomers, and solvates.

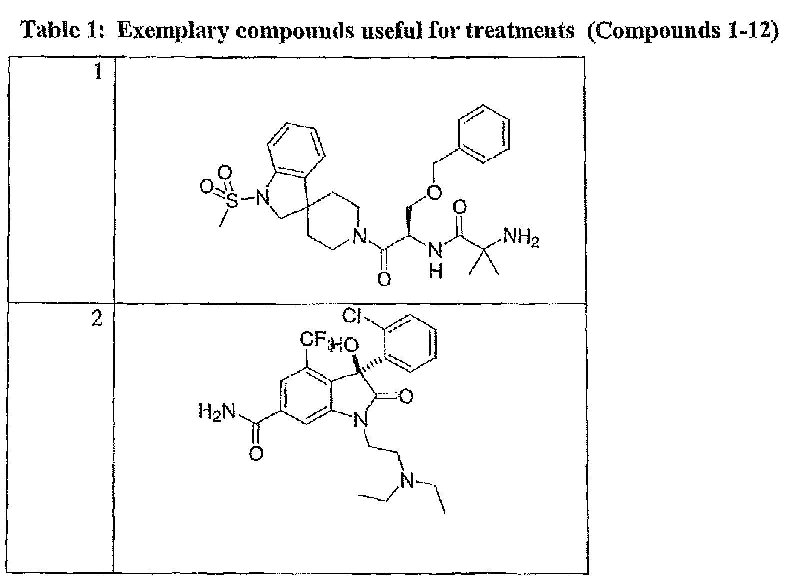

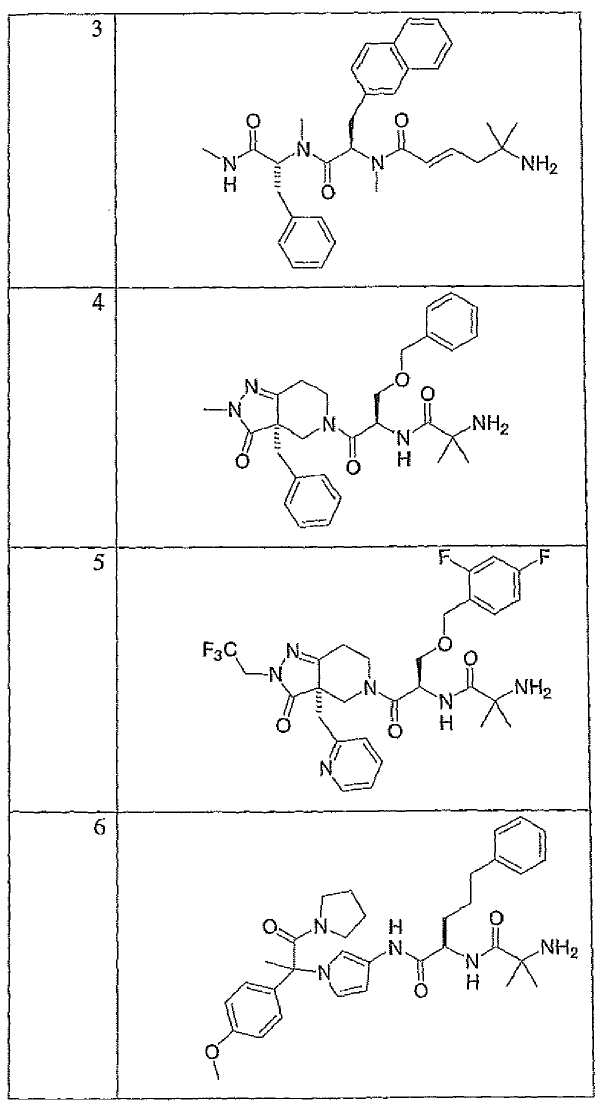

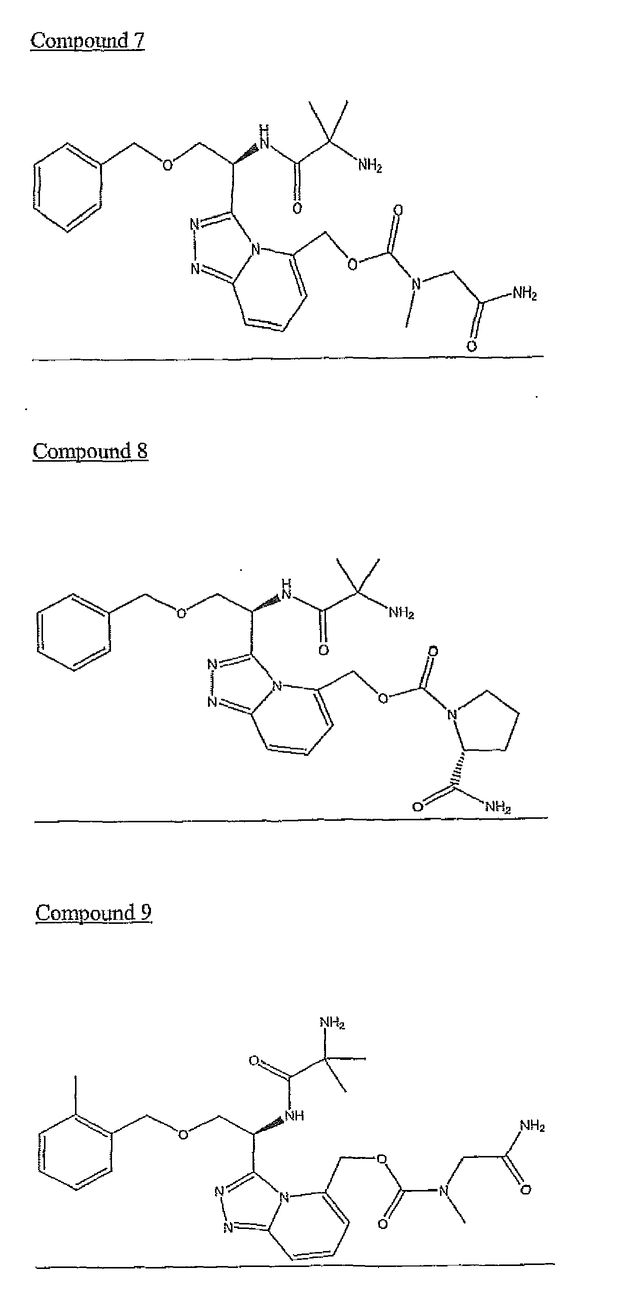

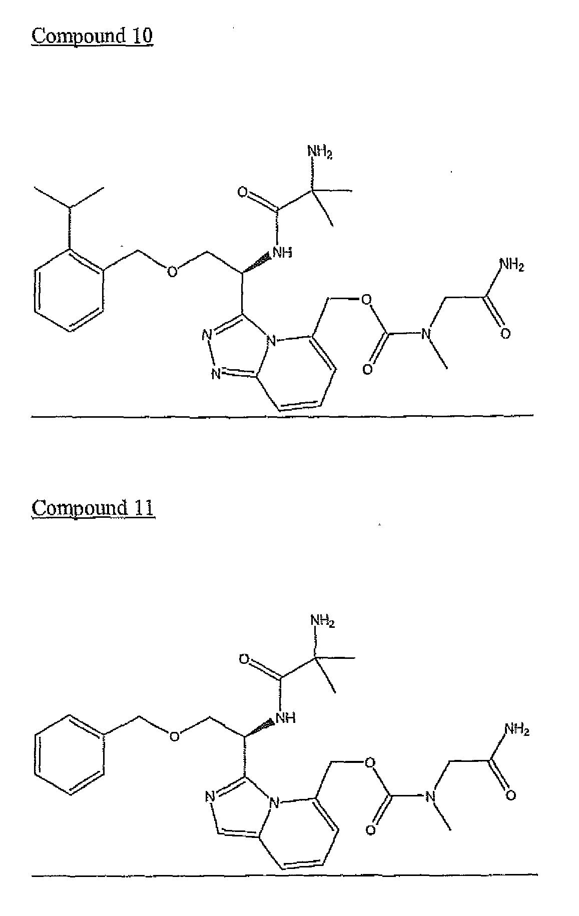

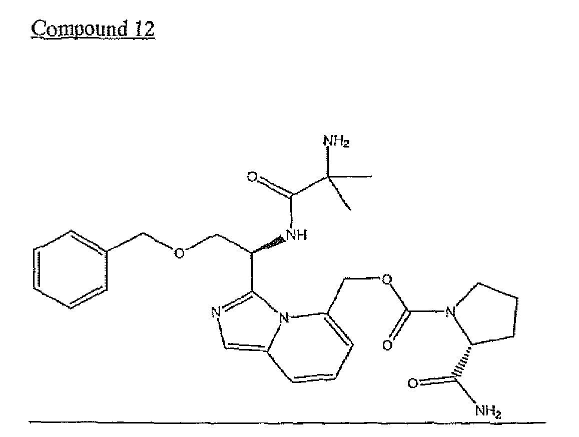

Although the compounds of Table 1 are preferred, related compounds can also be used to treat or prevent a disorder described herein, e.g., ileus or gastroparesis.

Related compounds include those described in U.S. Patent No. 5,578,593, and in particular those described from col. 2, line 20 to col.13, line 24, U.S. Patent No. 5,652,235, and in particular those described from col. 2, line 16 to col. 10, line 20, and U.S. Patent No. 6,420,376, and in particular those described from col. 2, line 26 to col. 21, line 45. These patents, including the cited sections, are hereby incorporated by reference. These compounds are related to compound 1 of Table 1.

Related compounds include those described in U.S. Patent No. 6,576,656, and in particular those described from col. 2, line 5 to col. 4, line 42. This patent, including the cited section, is hereby incorporated by reference. These compounds are related to compound 2 of Table 1.

Related compounds include those described in U.S. Patent Publication No. US2004/0063636, and in particular those described from p. 5, [0086] to p. 6, including, e.g., NNC 26-1291, NNC 26-1187, NN 703, and U.S. Patent No. 6,350,767, and in particular those described from col. 2, line 1 to col. 8, line 65. These patent references, including the cited sections, are hereby incorporated by reference. These compounds are related to compound 3 of Table 1.

Related compounds include those described in U.S. Patent No. 6,482,825, and in particular those described from col. 3, line 2 to col. 20, line 15. This patent, including the cited section, is hereby incorporated by reference. These compounds are related to compounds 4 and 5 of Table 1.

Related compounds include those described in U.S. Patent No. 5,773,441, and in particular those described from col. 2, line 65 to col. 4, line 55, U.S. Patent No. 6,639,076, and in particular those described from col. 3, line 28 to col. 15, line 19, and WO 03/087070, and in particular those described from p. 3, line 33 to p. 7, line 33. These patent references, including the cited sections, are hereby incorporated by reference. These compounds are related to compound 6 of Table 1.

Compounds related to compounds 7-12 are described in WO 2004/021984, which is hereby incorporated by reference.

Other compounds can be used to treat one or more disorders described herein, in particular ileus or gastroparesis, including the compounds described in WO 2004/021984, and in particular those described from p. 2, line 13 to p. 16, line 2, WO 2000/54729, and in particular those described from p. 2, line 2 to page 6, line 12, WO 2001/85695, and in particular those described from p. 3, line 6 to p. 6, line 9, WO 2000/24398 and in particular those described from p. 2, line 1 to p. 6, line 9, WO 2003/104255, and in particular those described from p. 2, line 15 to p. 7, line 10, WO 03/081258, and in particular those described from p. 2, line 13 to p. 5, line 28. These patent references, including the cited sections, are hereby incorporated by reference.

Still other compounds the can be used to treat one or more disorders described herein, in particular ileus or gastroparesis, including those compounds disclosed in WO 2001/25257, and in particular those described from p. 4, line 15 to p. 9, line 7. This patent reference, including the cited section, is hereby incorporated by reference.

The compounds, for example, can bind to and agonize (e.g., activate) the ghrelin receptor (which, for example, stimulates the production/release of GH) or GH receptor, bind to and agonize the GHRH receptor, function as a somatostatin antagonist, function as a GH secretagogue agonist, or affect the GH/IGF-1 axis in any way such that the compound, when given at its appropriate dosage increases GH, IGF-1 levels and/or IGF-1 receptor signaling in the subject by at least 30% (e.g., 30, 35,40,45,50,55,60,65,70,75, or 80%). In another embodiment, the compound is administered at regular intervals (e.g., daily, weekly, biweekly, or monthly). In yet another embodiment, the compound is administered at regular intervals for at least two months (e.g., at least six or nine months or for at least one, two, five, ten, 20, 25, or 30 years). The method can include other features described herein.

In other embodiments, the compounds can be used to treat cachexia, particularly cancer related cachexia. An effective amount of compound described or referred to herein can be administered to a subject suffering from at risk of cachexia in the treatment or prevention of cachexia.

In still other embodiments, the compounds can be used as follows: stimulating growth hormone release in elderly humans; treating growth hormone deficient adults; prevention of catabolic side effects of glucocorticoids; treatment of osteoporosis; stimulation of the immune system, acceleration of wound healing; accelerating bone fracture repair; treatment of growth retardation; treating acute or chronic renal failure or insufficiency; treatment of physiological short stature, including growth hormone deficient children; treating short stature associated with chronic illness; treating growth retardation associated with obesity; treating growth retardation associated with Prader-Willi syndrome and Turner's syndrome; accelerating the recovery and reducing hospitalization of burn patients or following major surgery such as gastrointestinal surgery; treatment of intrauterine growth retardation, and skeletal dysplasia; treatment of hypercortisonism and Cushing's syndrome; treatment of peripheral neuropathies; replacement of growth hormone in stressed patients; treatment of osteochondrody-splasias, Noonans syndrome, sleep disorders, schizophrenia, depression, Alzheimer's disease, delayed wound healing, and psychosocial deprivation; treatment of pulmonary dysfunction and ventilator dependency; prevention or treatment of congestive heart failure, improving pulmonary function, restoring systolic and diastolic function, increasing myocardial contractility, decreasing peripheral total vascular resistance, diminishing or preventing loss of body weight and enhancing recovery following congestive heart failure; increasing appetite; attenuation of protein catabolic response after a major operation; treating malabsorption syndromes; reducing protein loss due to chronic illness such as cancer or AIDS; accelerating weight gain and protein accretion in patients on TPN (total parenteral nutrition); treatment of hyperinsulinemia including nesidioblastosis; adjuvant treatment for ovulation induction and to prevent and treat gastric and duodenal ulcers; stimulation of thymic development and prevention of the age-related decline of thymic function; adjunctive therapy for patients on chronic hemodialysis; treatment of immunosuppressed patients and to enhance antibody response following vaccination; increasing the total lymphocyte count of a human, in particular, increasing the T₄/T₈ cell ratio in a human with a depressed T₄/T₈ cell ratio resulting, for example, from infection, such as bacterial or viral infection, especially infection with the human immunodeficiency virus; treatment of syndromes manifested by non-restorative sleep and musculoskeletal pain, including fibromyalgia syndrome or chronic fatigue syndrome; improvement in muscle strength, mobility, maintenance of skin thickness, metabolic homeostasis, renal homeostasis in the frail elderly; stimulation of osteoblasts, bone remodelling, and cartilage growth; prevention and treatment of congestive heart failure; protection of cardiac structure and/or cardiac function; enhancing of recovery of a mammal following congestive heart failure; enhancing and/or improving sleep quality as well as the prevention and treatment of sleep disturbances; enhancing or improving sleep quality by increasing sleep efficiency and augmenting sleep maintenance; prevention and treatment of mood disorders, in particular depression; improving mood and subjective well being in a subject suffering from depression; stimulation of the immune system in companion animals and treatment of disorders of aging in companion animals; growth promotion in livestock; and stimulation of wool growth in sheep. Further, the compounds described herein are useful for increasing feed efficiency, promoting growth, increasing milk production and improving the carcass quality of livestock. In general, the compounds described herein are useful in a method of treatment of diseases or conditions which are benefited by the anabolic effects of enhanced growth hormone levels and/or benefited by increased gastrointestinal motility (e.g., by modulation of myenteric nerve activity) that comprises the administration of a compound described herein.

In particular, the compounds are useful in the prevention or treatment of a condition selected from the group consisting of: osteoporosis; catabolic illness; immune deficiency, including that in individuals with a depressed T₄ /T₈ cell ratio; bone fracture, including hip fracture; musculoskeletal impairment in the elderly; growth hormone deficiency in adults or in children; short stature in children; obesity; sleep disorders; protein loss due to chronic illness such as AIDS or cancer; and treating patients recovering from major surgery, wounds or burns, in a patient in need thereof.

In addition, the compounds may be useful in the treatment of illnesses induced or facilitated by corticotropin releasing factor or stress- and anxiety-related disorders, including stress-induced depression and headache, abdominal bowel syndrome, immune suppression, HIV infections, Alzheimer's disease, gastrointestinal disease, anorexia nervosa, hemorrhagic stress, drug and alcohol withdrawal symptoms, drug addiction, and fertility problems.

Other aspects described herein relate to a composition having a compound described or referred to herein and a pharmaceutically acceptable carrier; or a compound described or referred to herein, an additional therapeutic compound (e.g., a mu opiod receptor antagonist such as alvimopan or methylnaltrexane or a motilin agonist such as erythromycin), and a pharmaceutically acceptable carrier. In some instances, a compound modulating the myenteric nerves and/or mesenteric nervous system (e.g., a compound described in Table 1) has a synergistic effect with the mu opioid receptor antagonist and thus can be co-administered for an increased therapeutic effect. Examples of additional therapeutic compounds that can be used in combination with a compound described or referred to herein include: a mu opioid receptor antagonist (e.g., alvimopan and methylmoltrexane), a motilin receptor agonist (e.g., erythromycin), a CIC-2 chloride channel activator (e.g., Lubiprostone), a dopamine antagonist (e.g., metoclopramide), a stimulant (e.g., glucocorticoid, progestational agents (e.g., menace), dronabinol, and cyproheptadine hydrochloride), and a cytokine blocker (e.g., hydrazine sulfate,pentoxifyline, thalidomide, melatonin,eicosapentaenoic acid and NSAIDS).

The details of one or more embodiments of the invention are set forth in the description below. Other features, objects, and advantages of the invention will be apparent from the description and from the claims. All patents, patent applications, and references mentioned herein are incorporated by reference in their entireties.

The compounds described herein can be used for a variety of purposes, e.g., therapeutic and diagnostic purposes. Many of the compounds increase GH e.g., in a subject, by inducing the production or secretion of GH. In some instances, the compounds agonize the ghrelin receptor.

Representative compounds of the invention are depicted in Table 1 and in other compounds described in the patent references incorporated by reference herein.

Compounds may be conveniently screened for growth hormone secretagogue activity. An exemplary assay may employ pituitary cells (e.g., rat pituitary cells) established in culture. The cells are contacted with the various compounds, and the levels of growth hormone secreted by the cells are determined accordingly. Growth hormone levels may be calculated using various radioimmunoassay techniques known to those of skill in the art. Screening of compounds for growth hormone secretagogue activity may conveniently be scaled up for high throughput screening. An exemplary assay for GHS-R binding is described, e.g., in Hansen et al. (1999) Eur. J. Endocrinol. 141:180-189.
Compounds may also be screened for their ability to modulate myenteric and/or mysenteric nerve activity through ex vivo electrophysiological monitoring of nerve network preparations. For example, the effect of putative agonists on presynaptic potentiation of acetylcholine or other neurotransmitter release can be monitored.

The compounds described herein, including the compounds of formulae described herein, are defined to include pharmaceutically acceptable derivatives and prodrugs thereof. A "pharmaceutically acceptable derivative and prodrug" means any pharmaceutically acceptable salt, ester, salt of an ester, or other derivative of a compound described herein (for example, an imidate ester of an amide), which, upon administration to a recipient, is capable of providing (directly or indirectly) the therapeutic activity of a compound described herein. Particularly favored derivatives and prodrugs are those that increase the bioavailability of the compounds described herein when such compounds are administered to a mammal (e.g., by allowing an orally administered compound to be more readily absorbed into the blood) or which enhance delivery of the parent compound to a biological compartment (e.g., the brain or lymphatic system) relative to the parent species. Preferred prodrugs include derivatives where a group which enhances aqueous solubility or active transport through the gut membrane is appended to the structure of formulae described herein.

The compounds described herein may be modified by appending appropriate functionalities to enhance selective biological properties. Such modifications are known in the art and include those which increase biological penetration into a given biological compartment (e.g., blood, lymphatic system, central nervous system), increase oral availability, increase solubility to allow administration by injection, alter metabolism and alter rate of excretion.

Pharmaceutically acceptable salts of the compounds described herein include those derived from pharmaceutically acceptable inorganic and organic acids and bases. Examples of suitable acid salts include acetate, adipate, benzoate, benzenesulfonate, butyrate, citrate, digluconate, dodecylsulfate, formate, fumarate, glycolate, hemisulfate, heptanoate, hexanoate, hydrochloride, hydrobromide, hydroiodide, lactate, maleate, malonate, methanesulfonate, 2-naphthalenesulfonate, nicotinate, nitrate, palmoate, phosphate, picrate, pivalate, propionate, salicylate, succinate, sulfate, tartrate, tosylate and undecanoate. Salts derived from appropriate bases include alkali metal (e.g., sodium), alkaline earth metal (e.g., magnesium), ammonium and N-(alkyl)₄⁺ salts. Any basic nitrogen-containing groups of the compounds disclosed herein can be subjected to quaternization. Water or oil-soluble or dispersible products may be obtained by such quaternization.

Synthesis of compounds

The compounds can be made by any appropriate method, including methods known to those skilled in the art, e.g., a method described in a patent references mentioned herein. Synthetic chemistry transformations and protecting group methodologies (protection and deprotection) useful in synthesizing the compounds described herein are known in the art and include, for example, those such as described in R. Larock, Comprehensive Organic Transformations, VCH Publishers (1989); T.W. Greene and P.G.M. Wuts, Protective Groups in Organic Synthesis, 2d. Ed., John Wiley and Sons (1991); L. Fieser and M. Fieser, Fieser and Fieser's Reagents for Organic Synthesis, John Wiley and Sons (1994); and L, Paquette, ed., Encyclopedia of Reagents for Organic Synthesis, John Wiley and Sons (1995), and subsequent editions thereof. Additionally, the compounds disclosed herein can be prepared on a solid support or using a solid phase peptide synthesis.

The compounds may also be prepared from a variety of substituted natural and unnatural amino acids. The preparation of many of these acids is described in U.S. Pat. No. 5,206,237. The preparation of these intermediates in racemic form is accomplished by classical methods familiar to those skilled in the art (Williams, R. M. "Synthesis of Optically Active α-Amino Acids" Pergamon Press: Oxford, 1989; Vol. 7). Several methods exist to resolve amino acids. One of the common methods is to resolve amino or carboxyl protected intermediates by crystallization of salts derived from optically active acids or amines. Alternatively, the amino group of carboxyl protected intermediates may be coupled to optically active acids. Separation of the individual diastereomers either by chromatographic techniques or by crystallization followed by hydrolysis of the chiral amide furnishes resolved amino acids. Similarly, amino protected intermediates may be converted to a mixture of chiral diastereomeric esters and amides. Separation of the mixture using methods described above and hydrolysis of the individual diastereomers provides (D) and (L) amino acids. Finally, an enzymatic method to resolve N-acetyl derivatives of (DL)-amino acids has been reported by Whitesides and coworkers in J. Am. Chem. Soc. 1989,111,6354-6364.

When it is desirable to synthesize these intermediates in optically pure form, established methods include: (1) asymmetric electrophilic amination of chiral enolates (J. Am. Chem. Soc. 1986, 108,6394-6395,6395-6397, and 6397-6399), (2) asymmetric nucleophilic amination of optically active carbonyl derivatives, (J. Am. Chem. Soc. 1992,114,1906; Tetrahedron Lett.1987, 28, 32), (3) diastereoselective alkylation of chiral glycine enolate synthons (J. Am. Chem. Soc. 1991, 113, 9276; J. Org. Chem. 1989, 54, 3916), (4) diastereoselective nucleophilic addition to a chiral electrophilic glycinate synthon (J. Am. Chem. Soc. 1986, 108, 1103), (5) asymmetric hydrogenation of prochiral dehydroamino acid derivatives ("Asymmetric Synthesis, Chiral Catalysis; Morrison, J. D., Ed; Academic Press: Orlando, Fla., 1985; Vol 5); and (6) enzymatic syntheses (Angew. Chem. Int. Ed. Engl. 1978, 17, 176).

Administration of compounds and formulations

The compounds can be administered to animals, including man, e.g., to increase gastrointestinal motility (e.g., by modulating myenteric nerve activity), to release growth hormone or otherwise increase growth hormone activity *in vivo.* In addition, these compounds can be administered to humans in vivo as a diagnostic tool to determine whether the pituitary is capable of releasing growth hormone. Serum samples taken before and after such administration can be assayed for growth hormone. Comparison of the amounts of growth hormone in each of these samples would be a means for directly determining the ability of the patient's pituitary to release growth hormone. Patient IGF-1 levels can also be monitored to complement analysis of growth hormone release. IGF-1 levels provide a temporally integrated assessment of the effect of a ghrelin agonist on the GH axis.

The compounds of the formulae described herein can, for example, be administered by injection, intravenously, intraarterially, subdermally, intraperitoneally, intramuscularly, or subcutaneously, orally, buccally, intranasally, transmucosally, or by inhalation. In general, doses range from about 0.001 to about 100 mg/kg of body weight, e.g., between 0.001-1 mg/kg, 1-100 mg/kg, or 0.01-5 mg/kg, every 4 to 120 hours, e.g., about every 6, 8, 12, 24, 48, or 72 hours, or according to the requirements of the particular compound and the particular disorder. In some preferred embodiments, the compounds are delivered intranasally or subcutaneously, for example, where the compounds are administered in the treatment of cachexia (e.g., cancer cachexia), gastroparesis, ileus, or lipodystrophy (e.g., HIV lipodystrophy).

Additionally, the compounds described herein are well suited to formulation as sustained release dosage forms. The formulations can be so constituted that they release the active ingredient only or preferably in a particular part of the intestinal tract, possibly over a period of time. Such formulations would involve coatings, envelopes, or protective matrices which may be made from polymeric substances or waxes.

Typically, the pharmaceutical compositions described herein will be administered from about 1 to about 6 times per day, for example, the compounds can be administered about 1 to about 4 (e.g., 1, 2, 3, or 4) hours prior to meal time. Alternatively, the compounds can be administered as a continuous infusion. Such administration can be used as a chronic or acute therapy. The amount of active ingredient that maybe combined with carrier materials to produce a single dosage form will vary depending upon the subject treated and the particular mode of administration. A typical preparation will contain from about 5% to about 95% active compound (w/w). Alternatively, such preparations contain from about 20% to about 80% active compound.

Lower or higher doses than those recited above may be required. Specific dosage and treatment regimens for any particular patient will depend upon a variety of factors, including the activity of the specific compound employed, age, body weight, general health status, sex, diet, time of administration, rate of excretion, drug combination, the severity and course of the disease, condition or symptoms, the patient's disposition to the disease, condition or symptoms, and the judgment of the treating physician.

The methods herein can include administration of an effective amount of compound or compound composition to achieve a therapeutic or prophylactic effect (e.g., preventing or reducing the severity of post-operative ileus). In some instances, the compound is administered to a patient prior to, during, or subsequent to a surgical procedure, e.g., within 12, 24, or 48 hours before or after a surgical procedure. The compound can, in some instances, provide a prophylactic effect and reduce the incidence or severity of a post-operative disorder such as post-operative ileus. In instances where the compound is administered to treat an acute disorder, the dosage regime generally extends the length of the disorder. For example, where a patient is treated for post-operative ileus, the compound can be administered following the surgical procedure over a course of about 24 hours to about 4 days. As described above, in some instances, the compound can also be administered before the surgical procedure, providing a prophylactic benefit to the patient.

In some instances, the compounds can be especially beneficial to patients who are underweight prior to surgery or chemotherapy. Accordingly, administration of a compound inducing the production or release of neurotransmitters such as GH prior to surgery can also improve the patient's tolerance to and recovery from the surgical procedure.

In some instances, the compound is administered in the treatment of a chronic disorder such as diabetic gastroparesis. The compound can, for example, be administered using a dosage and formulation that mimics the preprandial rise of ghrelin. For example, the patient can be administered a compound inducing the production or secretion of GH before a meal, for example, about 3 hours to about 5 minutes before the meal, such as about 2.5 hours, 2 hours, about 1.5 hours, 1 hour, 45 minutes, 30 minutes, 15 minutes, etc.

In some embodiments, the compounds can be administered together with other therapeutic compounds. For example, in the case of HIV lipodystrophy, the compounds can be administered together as part of the HIV therapeutic regime. In some instances, the compounds can be formulated together with one or more therapeutic compounds (e.g., protease inhibitors, reverse transcriptase inhibitors, fusion inhibitors, etc.). In other instances, the compounds can be independently formulated. In some instances, the compounds can be combined with one or more lipid-lowering agents, for example, in patients having elevated LDL or VLDL.

In some embodiments, the compounds are formulated and/or administered to increase gastric absorption relative to intestinal absorption. For example, a patient suffering from cachexia can be administered a compound that increases gastric absorption as opposed to intestinal absorption. In some embodiments, the route of administration and/or dosage regime can also influence the relative amounts of gastric absorption and intestinal absorption. In some instances, the compounds can be administered with one or more appetite stimulants.

Upon improvement of a patient's condition, a maintenance dose of a compound, composition or combination described herein may be administered, if necessary. Subsequently, the dosage or frequency of administration, or both, may be reduced, as a function of the symptoms, to a level at which the improved condition is retained. Patients may, however, require intermittent treatment on a long-term basis upon any recurrence of disease symptoms.

Pharmaceutical compositions can include a compound of a formula described herein or a pharmaceutically acceptable salt thereof; and any pharmaceutically acceptable carrier, adjuvant or vehicle. In some instances, the compositions can include an additional therapeutic agent such as a mu opiod receptor antagonist or other therapeutic agent described herein. Alternate compositions include a compound described or referred to herein or a pharmaceutically acceptable salt thereof; and a pharmaceutically acceptable carrier, adjuvant or vehicle. The compositions can be made, e.g., by combining one or more compounds delineated herein with one or more carriers and, optionally, one or more additional therapeutic compounds delineated herein.

In another embodiment, compounds described herein can be administered in combination with other growth hormone secretagogues such as the growth hormone releasing peptides GHRP-6, GHRP-1 as described in U.S. Pat. Nos. 4,411,890 and publications WO 89/07110, WO 89/07111 and B-HT920 as well as hexarelin and GHRP-2 as described in WO 93/04081 or growth hormone releasing hormone (GHRH, also designated GRF) and its analogs or growth hormone and its analogs or somatomedins including IGF-1 and IGF-2 or a-adrenergic agonists such as clonidine or serotonin 5HTID agonists such as sumitriptan or agents which inhibit somatostatin or its release such as physostigmine and pyridostigmine. In some embodiments, the compounds maybe used in combination with growth hormone releasing factor, an analog of growth hormone releasing factor, IGF-1, or IGF-2.

When the compositions described herein comprise a combination of a compound of the described or referred to herein and one or more additional therapeutic or prophylactic agents, both the compound and the additional compound should be present at dosage levels of between about 1 to 100%, and more preferably between about 5 to 95% of the dosage normally administered in a monotherapy regimen. Additionally, combinations of a plurality of compounds described or referred to herein are also envisioned. As noted above, the additional compounds may be administered separately, as part of a multiple dose regimen, from the compounds described herein. Alternatively, those compounds may be part of a single dosage form, mixed together with the compounds described herein in a single composition.

The term "pharmaceutically acceptable carrier or adjuvant" refers to a carrier or adjuvant that may be administered to a patient, together with a compound described herein, and which does not destroy the pharmacological activity thereof and is nontoxic when administered in doses sufficient to deliver a therapeutic amount of the compound.

Pharmaceutically acceptable carriers, adjuvants and vehicles that may be used in the pharmaceutical compositions described herein include, but are not limited to, ion exchangers, alumina, aluminum stearate, lecithin, self-emulsifying drug delivery systems (SEDDS) such as d-α-tocopherol polyethyleneglycol 1000 succinate, surfactants used in pharmaceutical dosage forms such as Tweens or other similar polymeric delivery matrices, serum proteins, such as human serum albumin, buffer substances such as phosphates, glycine, sorbic acid, potassium sorbate, partial glyceride mixtures of saturated vegetable fatty acids, water, salts or electrolytes, such as protamine sulfate, disodium hydrogen phosphate, potassium hydrogen phosphate, sodium chloride, zinc salts, colloidal silica, magnesium trisilicate, polyvinyl pyrrolidone, cellulose-based substances, polyethylene glycol, sodium carboxymethylcellulose, polyacrylates, waxes, polyethylene-polyoxypropylene-block polymers, polyethylene glycol and wool fat. Cyclodextrins such as *α*-, *β*-, and γ-cyclodextrin, may also be advantageously used to enhance delivery of compounds of the formulae described herein.

In some cases, the pH of the formulation may be adjusted with pharmaceutically acceptable acids, bases or buffers to enhance the stability of the formulated compound or its delivery form.

Compound 1 and related compounds

In one embodiment, the compound is compound 1 of Table 1 or a related compound. For example, the compound is a spiropiperidines or a compound of following structural formulas I and II:

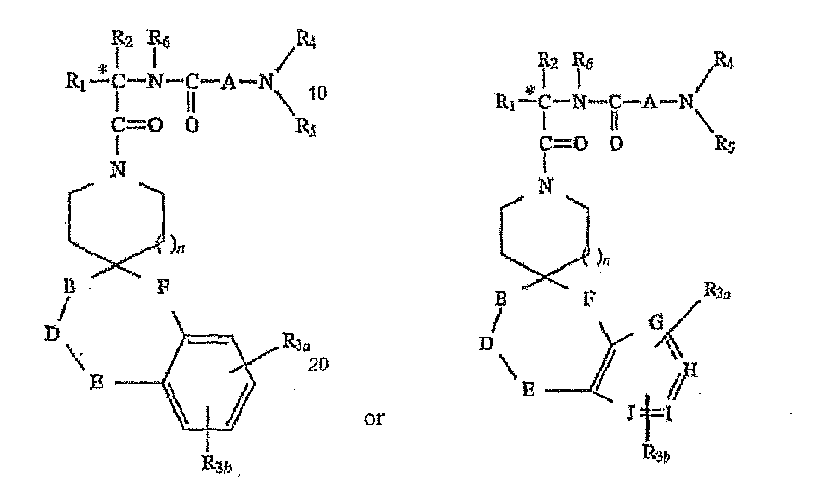

R₁, is C₁ -C₁0 alkyl, aryl, aryl (C₁ -C₆ alkyl) and C₃ -C₇ cycloalkyl (C₁-C₆ alkyl) or C₁ -C₅ alkyl-K-C₁ -C₅ alkyl, aryl(C₀ -C₅ alkyl)-K-(C₁ -C₅ alkyl), C₃ -C₇ cycloalkyl(C₀ -C₅ alkyl)-K-(C₁ -C₅ alkyl) where K is O, S(O)ₘ, N(R₂)C(O), C(O)N(R₂), OC(O), C(O)O, --CR₂ =CR2 - or --C≡C-- where the aryl groups are defined below and R₂ and the alkyl groups may be further substituted by 1-5 halogen, S(O)ₘ R₂a, 1 to 3 of OR₂a or C(O)OR₂a and the aryl groups maybe further substituted by phenyl, phenoxy, halophenyl, 1 to 3 of C₁ -C₆ alkyl, 1 to 3 of halogen, 1 to 2 of OR₂, methylenedioxy, S(O)ₘ R₂, 1 to 2 of CF₃, OCF₃, nitro, N(R₂)(R₂), N(R₂)C(O)(R₂), C(O)OR₂, C(O)N(R₂)(R₂), SO₂ N(R₂)(R₂), N(R₂)SO₂ aryl or N(R₂)SO₂ R₂;

R₂ is hydrogen, C₁ -C₆ alkyl, C₃ -C₇ cycloalkyl, and where two C₁ -C₆ alkyl groups are present on one atom, they may be optionally jointed to form a C₃ -C₈ cyclic ting optionally including oxygen, sulfur or NR₂a ; R₂a is hydrogen or C₁ -C₆ alkyl; R₃a and R₃b are independently hydrogen, halogen, C₁ -C₆ alkyl, OR₂, cyano, OCF₃, methylenedioxy, nitro, S(O)ₘ R, CF₃ or C(O)OR₂, and when R₃a and R₃b are in an ortho arrangement they can be joined to form a C₅ to C₈ aliphatic or aromatic ring optionally including 1 or 2 heteroatoms selected from oxygen, sulfur, or nitrogen;

R₄ and R₅ are independently hydrogen, C₁ -C₆ alkyl, substituted C₁ -C₆ alkyl where the substituents may be 1 to 5 halo, 1 to 3 hydroxy, 1 to 3 C₁ -C₁0 alkanoyloxy, 1 to 3 C₁ -C₆ alkoxy, phenyl, phenoxy, 2-furyl, C₁ -C₆ alkoxycarbonyl, S(O)ₘ (C₁ -C₆ alkyl); or R₄ and R₅ can be taken together to form --(CH₂)ᵣ Lₐ (CH₂)s-where Lₐ is C(R₂)₂, O, S(O)ₘ or N(R₂), r and s are independently 1 to 3 and R₂ is as defined above; R₆ is hydrogen or C₁ -C₆ alkyl; A is:

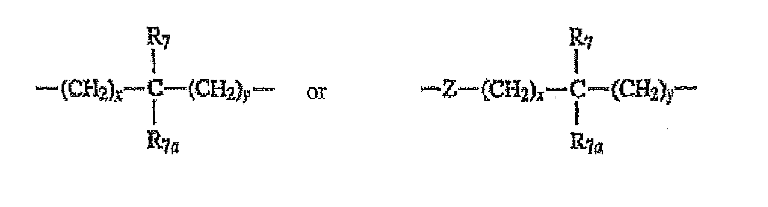

where x and y are independently 0-3; Z is N--R₂ or O; R₇ and R₇a are independently hydrogen, C₁ -C₆ alkyl, trifluoromethyl, phenyl, substituted C₁ -C₆ alkyl where the substituents are imidazolyl, phenyl, indolyl, p-hydroxyphenyl, OR₂, S(O)ₘ R₂, C(O)OR₂, C₃ -C₇ cycloalkyl, N(R₂)(R₂), C(O)N(R₂)(R₂); or R₇ and R₇a can independently be joined to one or both of R₄ and R₅ groups to form alkylene bridges between the terminal nitrogen and the alkyl portion of the R₇ or R₇a groups, wherein the bridge contains 1 to 5 carbons atoms;

B, D, E, and F are independently C(R₈)(R₁0), or C=O, such that one or two of B, D, E, or F may be optionally missing to provide a 5, 6, or 7 membered ring; or B and D or D and E taken together may be CR₈ =CR₁0, where CR₈ =CR₁0 may include a benzofusion in which R₈ and R₁0 ethylene units are linked to form a phenyl ring; R₈ and R₁0 are independently hydrogen, R₂, (CH₂)q aryl, (CH₂)q O(R₂), (CH₂)q O(CH₂)ₜ aryl, (CH₂)q OC(O)R₂, (CH₂)_{q} OC(O)(CH₂)ₜ aryl, (CH₂)q OC(O)N(R₂)(R₂), (CH₂)q OC(O)N(R₂)(CH₂)ₜ aryl, (CH₂)_{q} C(O)R₂, (CH₂)q C(O)(CH₂)ₜ aryl, (CH₂)q C(O)OR₂, (CH₂)_{q} C(O)O(CH₂)ₜ aryl, (CH₂)_{q} C(O)N(R₂)(R₂), (CH₂)_{q} C(O)N(R₂)(CH₂)ₜ aryl, (CH₂)_{q} N(R₂)(R₂), (CH₂)q N(R₂)(R₉), (CH₂)q S(O)ₘ R₂, (CH₂)_{q} S(O)ₘ (CH₂)ₜ aryl, (CH₂)q SO₂ N(R₂)(R₂), (CH₂)q SO₂ N(R₂)(CH₂)ₜ aryl, (CH₂)q (1H-tetrazol-5-yl), (CH₂)_{q} C(O)NHSO₂ R₂, (CH₂)_{q} C(O)NHSO₂ (CH₂)ₜ aryl, (CH₂)q SO₂ NHC(O)R₂, (CH₂)q SO₂ NHC(O)(CH₂)ₜ aryl, (CH₂)q SO₂ NH(CH₂)ₜ aryl, (CH₂)q SO₂ NH--CΞN and the (CH₂)ₜ may be substituted by 1 to 2 C₁-4 alkyl and the R₂, (CH₂)q and aryl groups may optionally be substituted by 1 to 5 halogen, 1 to 30R₂a, C(O)OR₂a, C(O)O(CH₂)ₜ aryl, 1 to 3 C₁-C₄ alkyl, C(O)N(R₂a)(R₂a), SO₂ N(R₂a)(R₂a), S(O)ₘ R₂a, N(R₂a)(R₂a), 1 to 2 CF₃, or 1H-tetrazol-5-yl; R₉ is R₂, (CH₂)q aryl, C(O)R₂, C(O)(CH₂)ₜ aryl, C(O)N(R₂)(R₂), C(O)N(R₂)(CH₂)ₜ aryl, C(O)OR₂, C(O)O(CH₂)t aryl, S(O)₂ N(R₂)(R₂), SO₂ N(R₂)(CH₂)ₜ aryl, SO₂ R₂ or SO₂ (CH₂)ₜ aryl and te (CH₂)ₜ may be substituted by 1 to 2 C₁ -C₄ alkyl and the R₂, (CH₂)q and aryl groups may optionally be substituted by 1 to 5 halogen, 1 to 3 OR₂a, C(O)OR₂a, C(O)O(CH₂)t aryl, 1 to 3 C₁ -C₄ alkyl, C(O)N(R₂a)(R₂a), SO₂ N(R₂a)(R₂a), S(O)ₘ R₂a, N(R₂a)(R₂a) or 1 to 2 CF₃; m is 0 to 2; n is 1 or 2; q is 0 to 3; t is 0 to 3; and G, H, I and J are carbon, nitrogen, sulfur or oxygen atoms, such that at least one is a heteroatom and one of G, H, I or J may be optionally missing to afford 5 or 6 membered heterocyclic aromatic rings; and pharmaceutically acceptable salts and individual diastereomers thereof.

Representative compounds include the following: N-[1(R)-[(2,3-Dihydrospiro[1H-indene-1,4'-piperidin]-1'-yl)carbonyl]-2-(1H -indol-3-yl)ethyl]-2-amino-2-methylpropanamide; N-[1(RS)-[(2,3-Dihydrospiro[1H-indene-1,4'-piperidin]-1'-yl)carbonyl]-2-(5 -fluoro-1H-indol-3-yl)ethyl]-2-amino-2-methylpropanamide; N-[1(RS)-[(2,3-Dihydro-3-oxospiro[1H-indene-1,4'-piperidin]-1'-yl)-carbonyl]-2-(1H-indol- 3-yl)ethyl]-2-amino-2-methylpropanamide; N-[1(RS)-[(2,3-Dihydro-3(RS)-hydroxyspiro[1H-indene-1,4'-piperidin]-1'-yl) carbonyl]-2-(1H-indol-3-yl)ethyl]-2-amino-2-methylpropanamide; N-[1(R)-[(2,3-Dihydro-6-fluorospiro[1H-indene-1,4'-piperidin]-1'-yl) carbonyl]-2-(indol-3-yl)ethyl]-2-amino-2-methylpropanamide;N-[1(R)-[(2,3-Dihydrospiro[1H-indene-1,4'-piperidin]-1'-yl)carbonyl]-2-(phenylmethyloxy)ethyl]-2-amino-2-methylpropanamide;N-[1(R)-[(2,3-Dihydro-3-oxospiro[1H-indene-1,4'-piperidin]-1'-yl)carbonyl] -2-(phenylmethyloxy)ethyl]-2-amino-2-methylpropanamide ; N-[1(R)-[(2,3-Dihydro-3(RS)-hydroxyspiro[1H-indene-1,4'-piperidin]-1'-yl) carbonyl]-2-(phenylmethyloxy)ethyl]-2-amino-2-methylpropanamide; N-[1(R)-[(2,3-Dihydrospiro[1H-indene-1,4'-piperidin]-1'-yl)carbonyl]-2-(2',6'-difluorophenylmethyloxy)ethyl]-2-amino-2-methylpropanamide; N-[1(RS)-[(2,3-Dihydro-3(RS)-hydroxyspiro[1H-indene-1,4'-piperidin]-1'-yl) carbonyl]-3-phenylpropyl]-2-amino-2-methylpropanamide; N-[1(R)-[(2,3-Dihydro-3-oxospiro[1H-indene-1,4'-piperidin]-1'-yl)carbonyl] -3-cyclohexylpropyl]-2-amino-2-methylpropanamide; N-[1(R)-[(2,3-Dihydro-3-(RS)-hydroxyspiro[1H-indene-1,4'-piperidin]-1'-yl) carbonyl]-3-cyclohexylpropyl]-2-amino-2-methylpropanamide ; N-[1(R)-[(2,3-Dihydro-3-oxospiro[1H-indene-1,4'-piperidin]-1'-yl)carbonyl] -4-phanylbutyl]-2-amino-2-methylpropanamide; N-[1(R)-[(2,3-Dihydro-3(RS)-hydroxyspiro[1H-indene-1,4'-piperidin]-1'-yl) carbonyl]-4-phenylbutyl]-2-amino-2-methylpropanamide; 1'-[2(R)-[(2-amino-2-methyl-1-oxopropyl)amino]-3-(indole-3-yl)-1-oxopropyl]-2,3-dihydrospiro[1H-indene-1,4'-piperidine]-3-carboxylic acid; 1'[2(R)-[(2-amino-2-methyl-1-oxopropyl)amino]-3-(indole-3-yl)-1-oxopropyl] -2,3-dihydrospiro[1H-indene-1,4'-piperidine-3-carboxylic acid ethyl ester; 1'-[2(R)-[(2-amino-2-methyl-1-oxopropyl)amino]-5-phenyl-1-oxopentyl]-2,3-dihydrospiro[1H-indene-1,4'-piperidine]-3-carboxylic acid; 1'-[2(R)-[(2-amino-2-methyl-1-oxopropyl)amino]-5-phenyl-1-oxopentyl]-2,3-dihydrospiro[1H-indene-1,4'-piperidine]-3-carboxylic acid ethyl ester; 1'-[2(R)-[(2-amino-2-methyl-1-oxopropyl)amino]-3-(indole-3-yl)-1-oxopropyl]-2,3-dihydrospiro[1H-indene-1,4'-piperidine]-3-acetic acid; 1'-[2(R)-[(2-amino-2-methyl-1-oxopropyl)amino]-3-(indole-3-yl)-1-oxopropyl]-2,3-dihydrospiro[1H-indene-1,4'-piperidine]-3(R)-acetic acid; 1'-[2(R)-[(2-amino-2-methyl-1-oxopropyl)amino]-3-(indole-3-yl)-1-oxopropyl]-2,3-dihydrospiro[1H-indene-1,4'-piperidine]-3(S)-acetic acid; 1'-[2(R)-[(2-amino-2-methyl-1-oxopropyl)amino]-3-(indole-3-yl)-1-oxopropyl]-2,3-dihydrospiro[1H-indene-1,4'-piperidine]-3-acetic acid ethyl ester; 1'-[2(R)-[(2-amino-2-methyl-1-oxopropyl)amino]-3-(indole-3-yl)-1-oxopropyl]-2,3-dihydrospiro[1H-indene-1,4'-piperidine]-3(R)-acetic acid ethyl ester; 1'-[2(R)-[(2-amino-2-methyl-1-oxopropyl)amino]-3-(indole-3-yl)-1-oxopropyl]-2,3-dihydrospiro[1H-indene-1,4'-piperidine]-3(S)-acetic acid ethyl ester; 1'-[2(R)-[(2-amino-2-methyl-1-oxopropyl)amino]-3-(5-fluoroindole-3-yl)-1-oxopropyl]-2,3-dihydrospiro[1H-indene-1,4-piperidine]-3-acetic acid; 1'-[2(R)-[(2-amino-2-methyl-1-oxopropyl)amino]-3-(5-fluoroindole-3-yl)-1-oxopropyl]-2,3-dihydrospiro[1H-indene-1,4-piperidine]-3(R)-acetic acid; 1'-[2(R)-[(2-amino-2-methyl-1-oxopropyl)amino]-3-(5-fluoroindole-3-yl)-1-oxopropyl]-2,3-dihydospiro[1H-indene-1,4'-piperidine]-3(S)-acetic acid; 1'-[2(R)-[(2-amino-2-methyl-1-oxopropyl)amino]-3-(5-fluoroindole-3-yl)-1-oxopropyl]-2,3-dihydrospiro[1H-indene-1,4'-piperidine]-3-acetic acid ethyl ester; 1'-[2(R)-[(2-amino-2-methyl-1-oxopropyl)amino]-3-(5-fluoroindole-3-yl)-1-oxopropyl]-2,3-dihydrospiro[1H-indene-1,4'-piperidine]-3(R)-acetic acid ethyl ester; 1'-[2(R)-[(2-amino-2-methyl-1-oxopropyl)amino]-3-(5-fluoroindole-3-yl)-1-oxopropyl]-2,3-dihydrospiro[1H-indene-1,4'-piperidine]-3(S)-acetic acid ethyl ester; 1'-[2(R)-[(2-amino-2-methyl-1-oxopropyl)amino]3-(phenylmethoxy)-1-oxopropyl]-2,3-dihydrospiro[1H-indene-1,4'-piperidine]-3-acetic acid; 1'-[2(R)-[(2-amino-2-methyl-1-oxopropyl)amino]-3-(phenylmethoxy)-1-oxopropyl]-2,3-dihydrospiro[1H-indene-1,4'-piperidine]-3-acetic acid ethyl ester; 1'-[2(R)-[(2-amino-2-methyl-1-oxopropyl)amino]-3-(2,6-difluorophenylmethoxy)-1-oxopropyl]-2,3-dihydrospiro[1H-indene-1,4'-piperidine]-3-acetic acid; 1'-[2(R)-[(2-amino-2-methyl-1-oxopropyl)amino]-3-(2,6-difluorophonylmethoxy)-1-oxopropyl]-2,3-dihydrospiro[1H-indene-1,4'-piperidine]-3-acetic acid ethyl ester; 1'-[2(R)-[(2-amino-2-methyl-1-oxopropyl)amino]-3-(5-fluoroindole-3-yl)-1-oxopropyl]-2,3-dihydro-6-fluorospiro[1H-indene-1,4'-piperidine]-3-acetic acid; 1'-[2(R)-[(2-amino-2-methyl-1-oxopropyl)amino]-3-(5-fluoroindole-3-yl)-1-oxopropyl]-2,3-dihydro-6-fluorospiro[1H-indene-1,4'-piperidine]-3-acetic acid ethyl ester; 1'-[2(R)-[(2-amino-2-methyl-1-oxopropyl)amino]-5-phenyl-1-oxopentyl]-2,3-dihydrospiro[1H-indene-1,4'-piperidine]-3-propanoic acid; 1'-[2(R)-[(2-amino-2-methyl-1-oxopropyl)amino]-5-phenyl-1-oxopentyl]-2,3-dihydrospiro[1H-indene-1,4'-piperidine]-3-propionic acid ethyl ester; 1'-[2(R)-[(2-amino-2-methyl-1-oxoptopyl)amino]-5-phenyl-1-oxopentyl]-2,3-dihydrospiro[1H-indene-1,4'-piperidine]-3-acetic acid; 1'-[2(R)-[(2-amino-2-methyl-1-oxopropyl)amino]-5-phenyl-1-oxopentyl]-2,3-dihydrospiro[1H-indene-1,4'-piperidine]-3(S)-acetic acid; 1'-[2(R)-[(2-amino-2-methyl-1-oxopropyl)amino]-5-phenyl-1-oxopentyl]-2,3-dihydrospiro[1H-indene-1,4'-piperidine]-3(R)-acetic acid; 1'-[2(R)-[(2-amino-2-methyl-1-oxopropyl)amino]-5-phenyl-1-oxopentyl]-2,3-dihydrospiro[1H-indene-1,4'-piperidine]-3-acetic acid ethyl ester; 1'-[2(R)-[(2-amino-2-methyl-1-oxopropyl)amino]-5-phenyl-1-oxopentyl]-2,3-dihydrospiro[1H-indene-1,4'-piperidine]-3(S)-acetic acid ethyl ester; 1'-[2(R)-[(2-amino-2-methyl-1-oxopropyl)amino]-5-phenyl-1-oxopentyl]-2,3-dihydrospiro[1H-indene-1,4'-piperidine]-3(R)-acetic acid ethyl ester; N-Ethyl-1'-[2(R)-[(2-amino-2-methyl-1-oxopropyl)amino]-5-phenyl-1-oxopentyl]-2,3-dihydrospiro[1H-indene-1,4'-piperidine]-3-acetamide; N-Ethyl-1'-[2(R)-[(2-amino-2-methyl-1-oxopropyl)amino]-5-phenyl-1-oxopentyl]-2,3-dihydrospiro[1H-indene-1,4'-piperidine]-3(S)-acetamide; N-Ethyl-1'-[2(R)-[(2-amino-2-methyl-1-oxopropyl)amino]-5-phenyl-1-oxopenty1]-2,3-dihydrospiro[1H-indene-1,4'-piperidine]-3-acetamide; 1'-[2(R)-[(2-amino-2-methyl-1-oxopropyl)amino]-5-phenyl-1-oxopentyl]-2,3-dihydro-6-fluorospiro[1H-indene-1,4'-piperidine]-3-acetic acid; 1'-[2(R)-[(2-amino-2-methyl-1-oxopropyl)amino]-5-phenyl-1-oxopentyl-2,3-di hydro-6-fluorospiro[1H-indene-1,4'-piperidine]-3-acetic acid ethyl ester; 1'-[2(R)-[(2-amino-2-methyl-1-oxopropyl)amino]-5-phenyl-1-oxopentyl]-2,3-dihydrospiro[1H-indene-1,4'-piperidine]-3-propanoic acid; 1'-[2(R)-[(2-amino-2-methyl-1-oxopropyl)amino]-5-phenyl-1-oxopentyl]-2,3-dihydrospiro[1H-indene-1,4'-piperidine]-3-propanoic acid ethyl ester; 1'-[2(R)-[(2-amino-2-methyl-1-oxopropyl)amino]-3-(indole-3-yl)-1-oxopropyl]-2,3-dihydro-6-fluorospiro[1H-indene-1,4'-piperidine]-3-acetic acid; and pharmaceutically acceptable salts and individual diastereomers (where unspecified) thereof.

Compound 2 and related compounds

In one embodiment, the compound is compound 2 of Table 1 or a related compound. Compounds related to compound 2 include an oxindole derivative of Formula III [1] or a prodrug thereof, or a pharmaceutically acceptable salt thereof: 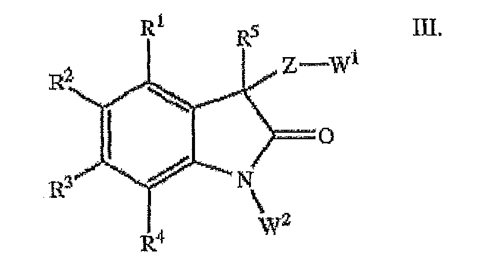 wherein R¹, R², R³ and R⁴ are the same or different and each is independently hydrogen, optionally substituted alkyl, optionally substituted alkenyl, optionally substituted alkynyl, optionally substituted aryl, optionally substituted heteroaryl, halogen, cyano, nitro, hydroxy, optionally substituted amino, alkoxy, alkanoyl, alkoxycarbonyl, optionally substituted sulfamoyl, optionally substituted carbamoyl, alkylthio, alkylsulfinyl, alkylsulfonyl, alkylsulfonylamino or alkanoylamino, provided that all of R¹, R², R³ and R⁴ are not simultaneously hydrogen;
R⁵ is optionally substituted aryl or optionally substituted heteroaryl; Z is --O-- or-NH--; one of W¹ and W² is hydrogen, alkyl or --Y--CON(R¹0)R¹1; the other of W¹ and W² is 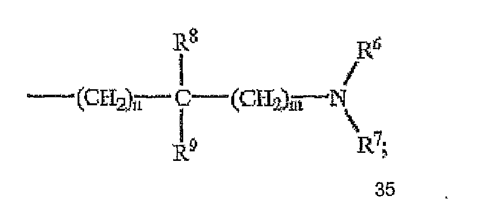 n is 1, 2 or 3; m is 0, 1, 2 or 3; Y is single bond or C₁ -C₃ alkylene;
R⁶ and R⁷ are the same or different and each is independently hydrogen, optionally substituted alkyl or optionally substituted cycloalkyl; or R⁶ and R⁷ are taken together with the adjacent nitrogen atom to form optionally substituted saturated heterocyclic ring;
R⁸ and R⁹ are the same or different and each is independently hydrogen or optionally substituted alkyl; or R⁸ and R⁹ are taken together with the adjacent carbon atom to form optionally substituted cycloalkane or optionally substituted saturated heterocyclic ring;
R⁸ and R⁶ may be taken together to form C₁ -C₅ alkylene in which case R⁷ is hydrogen, optionally substituted alkyl or optionally substituted cycloalkyl, and R⁹ is hydrogen or optionally substituted alkyl;
R¹0 and R¹ 1 are the same or different and each is independently hydrogen or alkyl; or R¹0 and R¹1 are taken together with the adjacent nitrogen atom to form optionally substituted saturated heterocyclic ring.
A second example [2] is an oxindole derivative or a prodrug thereof, or a pharmaceutically acceptable salt thereof according to [1] wherein R¹, R², R³ and R⁴ are independently hydrogen, alkyl optionally substituted by halogen, optionally substituted alkenyl, optionally substituted alkynyl, optionally substituted carbamoyl, halogen, cyano, nitro, alkanoyl, alkoxycarbonyl, alkylsulfinyl or alkylsulfonyl, provided that all of R¹, R², R³ and R⁴ are not simultaneously hydrogen.

A third example [3] is an oxindole derivative or a prodrug thereof, or a pharmaceutically acceptable salt thereof according to [1] wherein R¹, R², R³ and R⁴ are independently hydrogen, trifluoromethyl, carbamoyl, halogen, 4-carbamoyl-1-butynyl, 4-alkylcarbamoyl-1-butynyl, 4-dialkylcarbamoyl-1-butynyl, 4-morpholinocarbonyl-1-butynyl, --C ≡C--(CH₂)ₖ --Q, wherein k is 1 or 2; Q is hydroxy, alkylsulfonyl, alkanoylamino, alkylureido, 2-oxo-1-imidazolidinyl or 2-oxo-1,3-oxazolin-3-yl, provided that all of R¹, R², R³ and R⁴ are not simultaneously hydrogen.

A fourth example [4] is an oxindole derivative or a prodrug thereof, or a pharmaceutically acceptable salt thereof according to [3] wherein both of R² and R⁴ are hydrogen.

A fifth example [5] is an oxindole derivative or a prodrug thereof, or a pharmaceutically acceptable salt thereof according to [1] wherein both of R² and R⁴ are hydrogen; R¹ is trifluoromethyl, chlorine or bromine; and R³ is carbamoyl, halogen, 4-carbamoyl-1-butynyl, 4-alkylcarbamoyl-1-butynyl, 4-dialkylcarbamoyl-1-butynyl, 4-morpholinocarbonyl-1-butynyl, --C ≡C--(CH₂)ₖ --Q, wherein k and Q are as defined above.

A sixth example [6] is an oxindole derivative or a prodrug thereof, or a pharmaceutically acceptable salt thereof according to [1] wherein both of R² and R⁴ are hydrogen; R¹ is trifluoromethyl, chlorine or bromine; and R³ is carbamoyl. It is also possible to use an optical isomer of an oxindole derivative, of which the configuration at the C-3 position is equivalent to that of (+)-1-diethylaminoethyl-4-trifluoromethyl-6-carbamoyl-3-hydroxy-3-(2-chlorophenyl)oxindole, or a prodrug thereof, or a pharmaceutically acceptable salt thereof.

Specific examples of an oxindole derivative include: Specifically preferred examples of the oxindole derivatives are: 1-(2-Diethylaminoethyl)-4-trifluoromethyl-6-carbamoyl-3-hydroxy-3-(2-chloro-3-pyridyl)oxindole, 1-(2-Diethylaminoethyl)-4-trifluoromethyl-6-carbamoyl-3-hydroxy-3-(2-chloro-3-thienyl)oxindole, 1-(2-Diethylaminoethyl)-4-trifluoromethyl-6-carbamoyl-3-hydroxy-3-(5-indoly1)oxindole, 1-(2-Diethylaminoethyl)-4-trifluoromethyl-6-[3-(3-methyl-2-oxo-1-imidazolidinyl)-1-propynyl]-3-hydroxy-3-(2-chlorophenyl)oxindole, 1-(2-Diethylaminoethyl)-4-trifluoromethyl-6-[4-(3-methyl-2-oxo-1-imidazolidinyl)-1-butynyl]-3-hydroxy-3-(2-chlorophenyl)oxindole, 1-(2-Diethylaminoethyl)-4-trifluoromethyl-6-[4-dimethylcarbamoyl-1-butynyl] -3-hydroxy-3-(2-chlorophenyl)oxindole, 1-(2-Diethylaminoethyl)-4-trifluoromethyl-6-[5-dimethylcarbamoyl-1-pentynyl]-3-hydroxy-3-(2-chlorophenyl)oxindole, 1-(2-Diethylaminoethyl)-4-trifluoromethyl-6-dimethylcarbamoylethynyl-3-hydroxy-3-(2-chlorophenyl)oxindole, 1-(2-Diethylaminoethyl)-4-trifluoromethyl-6-carbamoylethynyl-3-hydroxy-3-(2 -chloro-4-bromophenyl)oxindole, 1-(2-Diethylaminoethyl)-4-trifluoromethyl-6-(2-carbamoylethenyl)-3-hydroxy- 3-(2-chloro-4-bromophenyl)oxindole, 1-(2-Diethylaminoethyl)-4-trifluoromethyl-6-(2-carbamoylethyl)-3-hydroxy-3- (2-chloro-4-bromophenyl)oxindole, 1-(2-Diethylaminoethyl)-4-trifluoromethyl-6-(4-amino-1-butynyl)-3-hydroxy-3 -(2-chlorophenyl)oxindole, 1-(2-Diethylaminoethyl)-4-trifluoromethyl-6-(4-acetamino-1-butynyl)-3-hydro xy-3-(2-chlorophenyl)oxindole, 1-(2-Diethylaminoethyl)-4-trifluoromethyl-6-(5-carboxy-1-pentynyl)-3-hydroxy-3-(2-chlorophenyl)oxindole, 1-(2-Diethylaminoethyl)-4-trifluoromethyl-6-sulfamoyl-3-hydroxy-3-(2-chloro phenyl)oxindole, 1-(2-Diethylaminoethyl)-4-trifluoromethyl-6-methylsulfamoyl-3-hydroxy-3-(2- chlorophonyl)oxindole, 1-(2-Diethylaminoethyl)-4-trifluoromethyl-6-dimethylsulfamoyl-3-hydroxy-3-(2-chlorophenyl)oxindole, 1-(2-Diethylaminoethyl)-4-trifluoromethyl-6-(4-sulfamoyl-1-butynyl)-3-hydroxy-3-(2-chlorophenyl)oxindole, 1-(2-Diethylaminoethyl)-4-trifluoromethyl-6-carbamoyl-3-hydroxy-3-(1-naphthyl)oxindole, 1-(2-Diethylaminoethyl)-4-trifluoromethyl-7-carbamoyl-3-hydroxy-3-(2-chlorophenyl)oxindole, 1-(2-Diethylaminoethyl)-6-carbanoyl-3-hydroxy-3-(2-chlorophenyl)oxindole, 1-(2-Diethylaminoethyl)-4-mothyl-6-carbamoyl-3-hydroxy-3-(2-chlorophenyl)oxindole, 1-(2-Diethylaminoethyl)-4-methoxy-6-carbamoyl-3-hydroxy-3-(2-chlorophenyl)oxindole, 1-(2-Diethylaminoethyl)-4-fluoro-6-carbamoyl-3-hydroxy-3-(2-chlorophenyl)oxindole, 1-(2-Diethylaminoethyl)-4-cyano-6-carbamoyl-3-hydroxy-3-(2-chlorophenyl)oxindole, 1-(2-Diethylaminoethyl)-4-hydroxy-6-carbamoyl-3-hydroxy-3-(2-chlorophenyl)oxindole, 1-(2-Diethylaminoethyl)-5-trifluoromethyl-6-carbamoyl-3-hydroxy-3-(2-chloro phenyl)oxindole, 1-(2-Diethylaminoethyl)-5-chloro-6-carbanloyl-3 -hydroxy-3-(2-chlorophenyl)oxindole, 1-(2-Diethylaminoethyl)-4-trifluoromethyl-5-chloro-6-carbamoyl-3-hydroxy-3- (2-chlorophenyl)oxindole, 1-(2-(2-Piperidinyl)ethyl-4-trifluoromethyl-6-carbamoyl-3-hydroxy-3-(2-chlorophenyl)oxindole, 1-(2-(2-Pyrrolidinyl)ethyl)-4-trifluoromethyl-6-carbamoyl-3-hydroxy-3-(2-chlorophenyl)oxindole, 1-(2-(N-methyl-2-pyrrolidinyl)ethyl)-4-trifluoromethyl-6-carbamoyl-3-hydroxy-3-(2-chlorophenyl)axindole, 1-(2-Piperidinylmethyl)-4-trifluoromethyl-6-carbamoyl-3-hydroxy-3-(2-chlorophenyl)oxindole, 1-(3-Amino-3-methylbutyl)-4-trifluoromethyl-6-carbamoyl-3-hydroxy-3-(2-chlorophenyl)oxindole, 1-(3-Aminobutyl)-4-trifluoromethyl-6-carbamoyl-3-hydroxy-3-(2-chlorophenyl) oxindole, 1-(4-Dimethylamino-3,3-dimethylbutyl)-4-trifluoromethyl-6-carbamoyl-3-hydroxy-3-(2-chlorophenyl)oxindole, 1-(2-Diethylaminoethyl)-4-trifluoromethyl-6-carbamoyl-3-hydroxy-3-(2,3-dichlorophenyl)oxindole, 1-(2-Diethylaminoethyl)-4-trifluoromethyl-6-carbamoyl-3-hydroxy-3-(2-chloro -4-methoxyphenyl)oxindole, 1-(2-Diethylaminoethyl)-4-trifluoromethyl-6-carbamoyl-3-hydroxy-3-(2-chloro -4-bromophenyl)oxindole, and 1-(2-Diethylaminoethyl)-4-trifluoromethyl-6-carbamoyl-3-hydroxy-3-(2,3,4-trichlorophenyl)oxindole.

Compounds 3 and Related Compounds

In one embodiment, the compound is compound 3 of Table 1 or a related compound. Representative compounds related to compound 3 include the following: 3-Aminomethyl-N-((1R, 2E, 4S)-4-carbamoyl-5-(2-naphthyl)-1-(2-naphthyl)methylpent-2-enyl)benzamide; Piperidine-4-carboxylic acid ((1R, 2E, 4S)-4-carbamoyl-5-(2-naphthyl)-1-(2-naphthyl)methylpent-2-enyl) amide; N-((1R)-1-((1R)-1-((1S)-5-Amino-1-(dimethylcarbamoyl)pentylcarbamoyl)-2-phenylethoxy)methyl-2-(2-naphthyl)ethyl)-3-aminomethylbenzamide; N-((1R, 4S)-4-(((1S)-5-Amino-1-(dimethylcarbamoyl)pentyl) carbamoyl)-1-((2-naphthyl)methyl)-2-oxo-5-phenylpentyl)-3-aminomethylbenzamide; N-((1R, 2R, 4S)-4-(((1S)-5-Amino-1-(dimethylcarbamoyl)pentyl) carbamoyl)-2-hydroxy-1-((2-naphthyl)methyl)-5-phenylpentyl)-3-aminomethylbenzamide: Piperidine-3-carboxylic acid ((1R, 2R, 4S)-4-(((1S)-5-amino-1-(dimethylcarbamoyl)pentyl) carbamoyl)-2-hydroxy-1-(2-naphthyl)methyl)-5-phenylpentyl)amide; 5-((1R)-1-(N-Methyl-N-((2R)-3-(2-naphthyl)-2-(piperidine-4-carbonylamino)propionyl)amino)-2-(2-naphthyl)ethyl)-[1,2,4]oxadiazole-3-carboxylic acid ethylester; 5-((1R)-1-(N-((2R)-2-(3-Aminomethylbenzoylamino)-3-(2-naphthyl)propionyl)-N-methylamino)-2-(2-naphthyl)ethyl)-[1,2,4]oxadiazole-3-carboxylic acid ethylester; 5-((1R)-1-(N-(2R)-2-(3-Aminomethylbenzoylamino)-3-(2-naphthyl)propionyl)-N-methylamino)-2-phenylethyl)-[1,3,4]oxadiazole-2-carboxylic acid amide. Additional examples include the following: 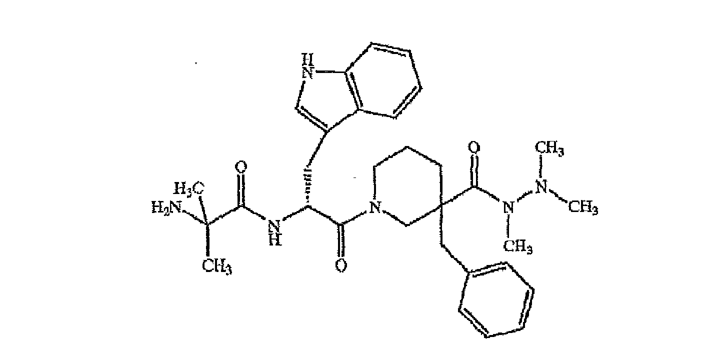 and 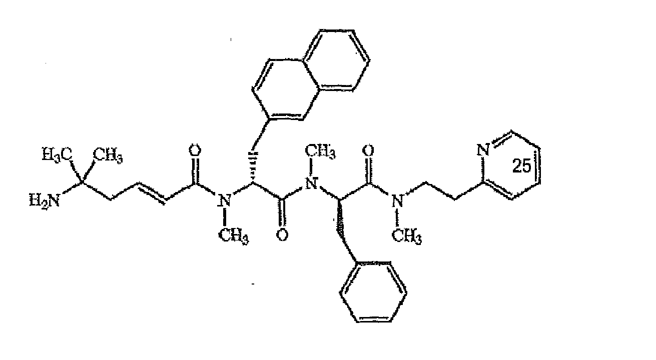 and

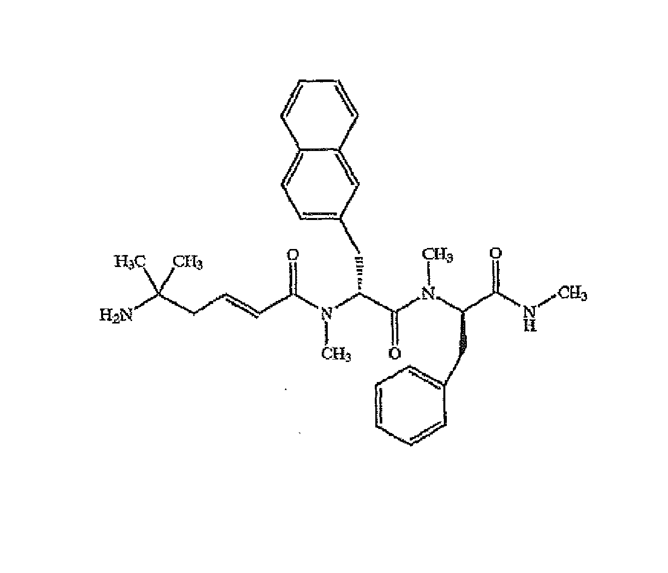

Compounds 4 and 5 and Related Compounds

In one embodiment, the compound is compound 4 or 5 of Table or a related compound. Exemplary compounds related to compounds 4 and 5 include compounds of the formula: 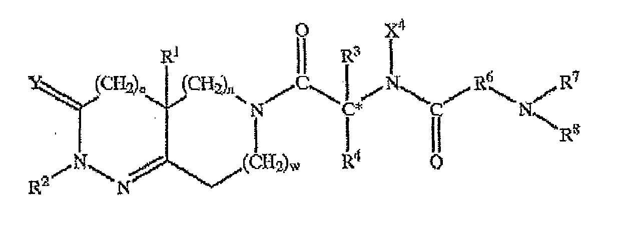 the racemic-diastereomeric mixtures and optical isomers of said compounds and the pharmaceutically-acceptable salts and prodrugs thereof,
Generally, e is 0 or 1; n and w are each independently 0, 1 or 2, provided that w and n cannot both be 0 at the same time; Y is oxygen or sulfur; R¹ is hydrogen, --CN,-(CH₂)q N(X⁶)C(O)X⁶, --(CH₂)q N(X⁶)C(O)(CH₂)ₜ --A¹, --(CH₂)q N(X⁶)SO₂ --(CH₂)ₜA¹, --(CH₂)q N(X⁶)SO₂ X⁶, --(CH₂)q N(X⁶)C(O)N(X⁶)(CH₂)ₜ --A¹, --(CH₂)_{q} N(X⁶)C(O)N(X⁶)(X⁶), --(CH₂)_{q} C(O)N(X⁶)(X⁶), --(CH₂)_{q} C(O)N(X⁶)(CH₂)ₜ --A¹,-(CH₂)_{q} C(O)OX⁶, --(CH₂)_{q} C(O)O(CH₂)ₜ --A¹, --(CH₂)_{q} OX⁶, --(CH₂)_{q} OC(O)X⁶,-(CH₂)_{q} OC(O)(CH₂)ₜ --A¹, --(CH₂)_{q} OC(O)N(X⁶)(CH₂)ₜ --A¹, --(CH₂)_{q} OC(O)N(X⁶)(X⁶), --(CH₂)_{q} C(O)X⁶, --CH₂)_{q} C(O)(CH₂)ₜ --A¹, --(CH₂)_{q} N(X⁶)C(O)OX⁶, --(CH₂)_{q} N(X⁶)SO₂ N(X⁶)(X⁶), --(CH₂)_{q} S(O)ₘ X⁶, --(CH₂)_{q} S(O)ₘ (CH₂)ₜ --A¹, --(C₁ -C₁0)alkyl, --(CH₂)ₜ --A¹, --(CH₂)_{q} --(C₃ -C₇)cycloalkyl, --(CH₂)_{q}-Y¹ --(C₁ -C₆)alkyl, --(CH₂)_{q} --Y¹ --(CH₂)ₜ --A¹ or --(CH₂)_{q} --Y¹ --(CH₂)ₜ --(C₃ - C₇)cycloalkyl; where the alkyl and cycloalkyl groups in the definition of R¹ are optionally substituted with (C₁ -C₄)alkyl, hydroxyl, (C₁ -C₄)alkoxy, carboxyl,-CONH₂, --S(O)ₘ (C₁ -C₆)alkyl, --CO₂ (C₁ -C₄)alkyl ester, 1 H-tetrazol-5-yl or 1, 2 or 3 fluoro; Y¹ is O, S(O)ₘ, --C(O)NX⁶ --, --CH=CH--, --C=C--, --N(X⁶)C(O)--,-C(O)N)X⁶ --, --C(O)O--, --OC(O)N(X⁶)--or --OC(O)--; q is 0,1,2,3 or4; t is 0, 1, 2 or 3; said (CH₂)_{q} group and (CH₂)ₜ group may each be optionally substituted with hydroxyl, (C₁ -C₄)alkoxy, carboxyl, --CONH₂, --S(O)ₘ (C₁ -C₆)akyl, --CO₂ (C₁ - C₄)alkyl ester, 1H-tetrazol-5-yl, 1, 2 or 3 fluoro, or 1 or 2 (C₁ -C₄)alkyl;

R² is hydrogen, C₁ -C₈)alkyl, --(C₀ -C₃)alkyl-(C₃ -C₈)cycloakyl, --(C₁ - C₄)alkyl-A¹ or A¹ ; where the alkyl groups and the cycloalkyl groups in the definition of R² are optionally substituted with hydroxyl, --C(O)OX⁶, --C(O)N(X⁶)(X⁶),-N(X⁶)(X⁶), --S(O)ₘ (C₁ -C₆)alkyl,--C(O)A¹, --C(O)(X⁶), CF₃, CN or 1, 2 or 3 halogen;

R³ is A¹, (C₁ -C₁O)alkyl, --(C₁ -C₆)alkyl-A¹, --(C₁ -C₆)alkyl-(C3 - C₇)cycloalkyl, --(C₁ -C₅)alkyl-X¹ --(C₁ -C₅)alkyl, --(C₁ -C₅)alkyl-X¹ --(C₀ -C₅)alkyl-A¹ or --(C₁ -C₅)alkyl-X¹ --(C₁ -C₅)alkyl-(C₃ -C₇)cycloalkyl; where the alkyl groups in the definition of R₃ are optionally substituted with, --S(O)ₘ (C₁ -C₆)alkyl, --C(O)OX³, 1, 2, 3, 4 or 5 halogens, or 1, 2 or 3 OX³ ; X¹ is O, S(O)ₘ, --N(X²)C(O)--,-C(O)N(X²)--, --OC(O)--, --C(O)O--, CX²=CX²--, --N(X²)C(O)O--, --OC(O)N(X²)-or --C ≡C--;

R⁴ is hydrogen, (C₁ -C₆)alkyl or (C₃ -C₇)cycloalkyl, or R⁴ is taken together with R³ and the carbon atom to which they are attached and form (C₅ - C₇)cycloalkyl, (C₅ --C₇)cycloalkenyl, a partially saturated or fully saturated 4- to 8-membered ring having 1 to 4 heteroatoms independently selected from the group consisting of oxygen, sulfur and nitrogen, or is a bicyclic ring system consisting of a partially saturated or fully saturated 5- or 6-membered ring, fused to a partially saturated, fully unsaturated or fully saturated 5- or 6-membered ring, optionally having 1 to 4 heteroatoms independently selected from the group consisting of nitrogen, sulfur and oxygen; X⁴ is hydrogen or (C₁ -C₆)alkyl or X⁴ is taken together with R⁴ and the nitrogen atom to which X⁴ is attached and the carbon atom to which R⁴ is attached and form a five to seven membered ring;

R⁶ is a bond or is 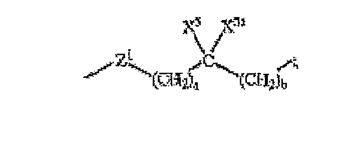 where a and b are independently 0, 1, 2 or 3; X⁵ and X⁵a are each independently selected from the group consisting of hydrogen, trifluoromethyl, A¹ and optionally substituted (C₁ -C₆)alkyl; the optionally substituted (C₁ -C₆)alkyl in the definition of X⁵ and X⁵a is optionally substituted with a substituent selected from the group consisting of A¹, OX², --S(O)ₘ (C₁ -C₆)alkyl, --C(O)OX², (C₃ -C₇)cycloalkyl,-N(X²)(X²) and --C(O)N(X²)(X²); or the carbon bearing X⁵ or X⁵a forms one or two alkylene bridges with the nitrogen atom bearing R⁷ and R⁸ wherein each alkylene bridge contains 1 to 5 carbon atoms, provided that when one alkylene bridge is formed then X⁵ or X⁵a but not both maybe on the carbon atom and R⁷ or R⁸ but not both may be on the nitrogen atom and further provided that when two alkylene bridges are formed then X⁵ and X⁵a cannot be on the carbon atom and R⁷ and R⁸ cannot be on the nitrogen atom; or X⁵ is taken together with X⁵a and the carbon atom to which they are attached and form a partially saturated or fully saturated 3- to 7-membered ring, or a partially saturated or fully saturated 4- to 8-membered ring having 1 to 4 heteroatoms independently selected from the group consisting of oxygen, sulfur and nitrogen; or X⁵ is taken together with X⁵a and the carbon atom to which they are attached and form a bicyclic ring system consisting of a partially saturated or fully saturated 5- or 6-membered ring, optionally having 1 or 2 heteroatoms independently selected from the group consisting of nitrogen, sulfur and oxygen, fused to a partially saturated, fully saturated or fully unsaturated 5- or 6-membered ring, optionally having 1 to 4 heteroatoms independently selected from the group consisting of nitrogen, sulfur and oxygen; Z¹ is a bond, O or N--X², provided that when a and b are both 0 then Z¹ is not N--X² or O; R⁷ and R⁸ are independently hydrogen or optionally substituted (C₁ -C₆)alkyl; where the optionally substituted (C₁ -C₆)alkyl in the definition of R⁷ and R⁸ is optionally independently substituted with A¹, --C(O)O--(C₁ -C₆)alkyl, --S(O)ₘ (C₁ -C₆)alkyl, 1 to 5 halogens, 1 to 3 hydroxy, 1 to 3 --O--C(O)(C₁ -C₁O)alkyl or 1 to 3 (C₁ -C₆)alkoxy; or

R⁷ and R⁸ can be taken together to form --(CH₂)ᵣ --L--(CH₂)ᵣ --; where L is C(X²)(X²), S(O)ₘ or N(X²); A¹ for each occurrence is independently (C₅ - C₇)cycloalkenyt, phenyl, or a partially saturated, fully saturated or fully unsaturated 4-to 8-membered ring optionally having 1 to 4 heteroatoms independently selected from the group consisting of oxygen, sulfur and nitrogen, a bicyclic ring system consisting of a partially saturated, fully unsaturated or fully saturated 5- or 6-membered ring, optionally having 1 to 4 heteroatoms independently selected from the group consisting of nitrogen, sulfur and oxygen, fused to a partially saturated, fully saturated or fully unsaturated 5- or 6-membered ring, optionally having 1 to 4 heteroatoms independently selected from the group consisting of nitrogen, sulfur and oxygen; A¹ for each occurrence is independently optionally substituted, in one or optionally both rings if A¹ is a bicyclic ring system, with up to three substituents, each substituent independently selected from the group consisting of F, C1, Br, I, OCF₃, OCF₂ H, CF₃, CH₃, OCH₃, --OX⁶, --C(O)N(X⁶)(X⁶), --C(O)OX⁶, oxo, (C₁ -C₆)alkyl, nitro, cyano, benzyl, --S(O)ₘ (C₁ -C₆)alkyl, 1H-tetrazol-5-yl, phenyl, phenoxy, phenylalkyloxy, halophenyl, methylenedioxy, --N(X⁶)(X⁶), --N(X⁶)C(O)(X⁶), --SO₂ N(X⁶)(X⁶),-N(X⁶)SO₂ -phenyl, --N(X⁶)SO₂ X⁶*,* --CONX¹1 X¹2, --SO₂ NX¹1 X¹2, --NX⁶ SO₂ X¹2, --NX⁶ CONX¹1 X¹2, --NX⁶ SO₂ NX¹1 X¹2, --NX⁶ C(O)X¹2, imidazolyl, thiazolyl or tetrazolyl, provided that if A¹ is optionally substituted with methylenedioxy then it can only be substituted with one methylenedioxy; where X¹1 is hydrogen or optionally substituted (C₁ -C₆)alkyl; the optionally substituted (C₁ -C₆)alkyl defined for X¹1 is optionally independently substituted with phenyl, phenoxy, (C₁ - C₆)alkoxycarbonyl, --S(O)ₘ (C₁ -C₆)alkyl 1 to 5 halogens, 1 to 3 hydroxy, 1 to 3 (C₁ - C₁O)alkanoyloxy or 1 to 3 (C₁ -C₆)alkoxy; X¹2 is hydrogen, (C₁ -C₆)alkyl, phenyl, thiazolyl, imidazolyl, furyl or thienyl, provided that when X¹2 is not hydrogen, X¹2 is optionally substituted with one to three substituents independently selected from the group consisting of Cl, F, CH₃, OCH₃, OCF₃ and CF₃ ; or X¹1 and X¹2 are taken together to form --(CH₂)ᵣ --L¹ -(CH₂)ᵣ --; L¹ is C(X²)(X²), O, S(O)ₘ or N(X²); r for each occurrence is independently 1, 2 or 3; X² for each occurrence is independently hydrogen, optionally substituted (C₁ -C₆)alkyl, or optionally substituted (C₃ - C₇)cycloalkyl, where the optionally substituted (C₁ -C₆)alkyl and optionally substituted (C₃ -C₇)cycloalkyl in the definition of X² are optionally independently substituted with -S(O)ₘ (C₁ -C₆)alkyl, --C(O)OX³, 1 to 5 halogens or 1 to 3 OX³ ; X³ for each occurrence is independently hydrogen or (C₁ -C₆)alkyl; X⁶ is independently hydrogen, optionally substituted (C₁ -C₆)alkyl, (C₂ -C₆)halogenated alkyl, optionally substituted (C₃ -C₇)cydoalkyl, (C₃ -C₇)--halogenated cycloalkyl, where optionally substituted (C₁ -C₆)alkyl and optionally substituted (C₃ -C₇)cycloalkyl in the definition of X⁶ is optionally independently substituted by 1 or 2 (C₁ -C₄)alkyl, hydroxyl, (C₁ -C₄)alkoxy, carboxyl, CONH₂, -S(O)ₘ (C₁ C₆)alkyl, carboxylate (C₁ - C₄)alkyl ester, or 1H-tetrazol-5-yl; or when there are two X⁶ groups on one atom and both X⁶ are independently (C₁ -C₆)alkyl, the two (C₁ -C₆)alkyl groups may be optionally joined and, together with the atom to which the two X⁶ groups are attached, form a 4- to 9-membered ring optionally having oxygen, sulfur or NX⁷ ; X⁷ is hydrogen or (C₁ -C₆)alkyl optionally substituted with hydroxyl; and m for each occurrence is independently 0, 1 or 2; with the proviso that: X⁶ and X¹2 cannot be hydrogen when it is attached to C(O) or S₂ in the form C(O)X⁶, C(O)X¹2, SO₂ X⁶ or SO₂ X¹2 ; and when R⁶ is a bond then L is N(X²) and each r in the definition --(CH₂)ᵣ --L--(CH₂)ᵣ -- is independently 2 or 3.

Compound 6 and related compounds

In one embodiment, the compound is compound 6 of Table 1 or a related compound, e.,g., a compound of Formula IV: 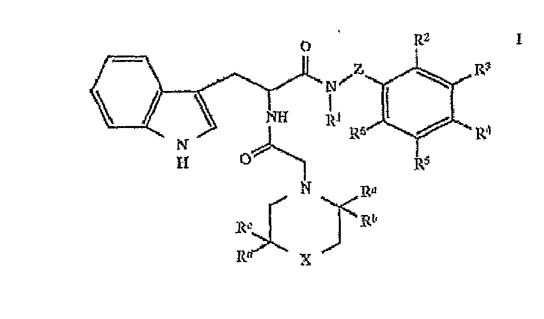 wherein: R¹ is hydrogen, C₁ -C₆ alkyl, or C₂ -C₆ alkanoyl; R², R³, R⁴, R⁵, and R⁶ are independently selected from the group consisting of hydrogen, halo, C₁ -C₆ alkyl, C₁ - C₆ alkoxy, C₁ -C₆ alkylamino, C₁ -C₆ alkylthio, amino, and trifluoromethyl;

R^{a} and R^{b} are each hydrogen or together form an oxo group; R^{c} and R^{d} are each hydrogen or together form an oxo group; Z is a bond or C₁ -C₆ alkylidenyl; and
X is

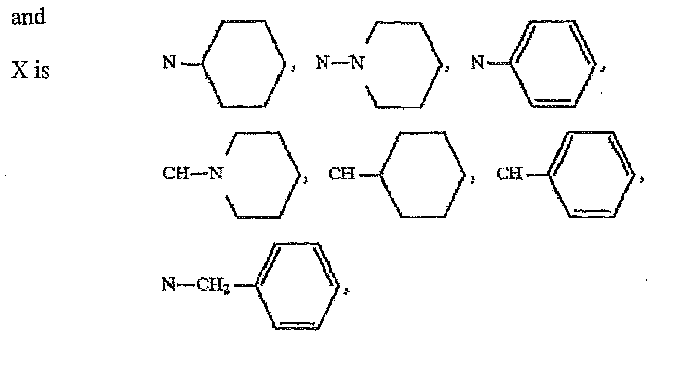

N--R⁷, CH--NR⁸ R⁹, or CH--R¹0 where R⁷, R⁸, R⁹, and R¹0 are independently selected from the group consisting of hydrogen and C₁ -C₆ alkyl; or a pharmaceutically acceptable salt or solvate thereof.

Compounds 7-12 and related compounds.

In one embodiment, the compound is compound 7, 8,9,10,11, or 12 of Table 1, or a heterocyclic aromatic compound having the general structure of formula V: 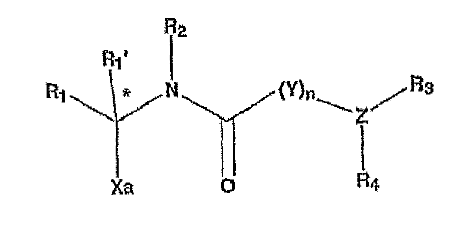 wherein Xa is 2 to 4 fused or spiro cycloalkyl, heterocycle, aryl or heteroaryl rings, wherein one or more of said rings may optionally be substituted with one to five substituents selected from the group consisting of Ra and Rb;

R₁ is a substituted or unsubstituted functional group selected from the group consisting of alkyl, aryl, alkenyl, alkynyl, arylalkyl, cycloalkyl, heterocycle, alkoxyalkyl, arylalkyloxyalkyl, aryloxyalkyl, hateroaryl, cycloalkylalkoxyalkyl, heteroarylalkoxy, heteroarylalkyl, heterocycloalkyl and heterocycloalkyl;

R₂, R₃ and R₄ are each independently a substituted or unsubstituted functional group selected from the group consisting of hydrogen, alkyl, alkenyl, alkynyl, aryl, arylalkyl, cycloalkyl, heterocycle, alkoxyalkyl, arylalkyloxyalkyl, aryloxyalkyl, heteroaryl, cycloalkylalkoxyalkyl, heteroarylalkyl and hetercycloalkyl, or R₃ and R₄ taken together can form a 3 to 8 membered cycloalkyl or heterocyclic ring, or one or more of R₃ and R₄ can be taken together with one or more of Y and Z to form a mono- or bicyclic cycloalkyl or heterocyclic ring;

R₁' is a substituted or unsubstituted functional group selected from the group consisting of hydrogen, alkyl, cycloalkyl, heterocycle, aryl and heteroaryl;

Y is a linking group selected from the group consisting of alkylene, alkenylene, alkynylene, arylene and heteroarylene, said linking group may optionally be substituted with one or more functional groups selected from the group consisting of alkyl, aryl, cycloalkyl, heterocycle, alkoxyalkyl, heteroaryl, arylalkyl, arylalkyloxyalkyl, aryloxyalkyl, cycloalkylalkoxyalkyl, heteroarylalkyl and heterocycloalkyl, halogen, --OR₅, --OC(O)R₅, --CF₃, --OCF₃, --N(R₅)C(O)R₅' and-NR₅R₅';

R₅ and R₅' for each occurrence are each independently selected from the group consisting of hydrogen, alkyl, cycloalkyl, heterocycle and aryl, wherein R₅ and R₅' for each occurrence may optionally be substituted with one or more Rb;

Ra and Rb for each occurrence are each independently selected from the group consisting of alkyl, alkenyl, alkynyl, halogen, cyano, carbonyl, --CN, aryl, arylalkyl, arylalkenyl, arylalkynyl, cycloalkyl, alkoxy, alkoxyalkyl, aryloxy, aryloxyalkyl, heterocycle, heteroaryl, heteroarylalkyl, --OR₂, --NR₅R₅', --CF₃,-SO₂R₆, --SO₂NR₆R₆', --(CH₂)ₘR₈ and R₉;

R₆ and R₆' for each occurrence are each independently selected from the group consisting of hydrogen, alkyl, alkenyl, alkynyl, alkylthioalkyl, alkoxyalkyl, aryl, arylalkyl, heterocycle, heteroaryl, heteroarylalkyl, heterocycloalkyl and cycloalkyl, wherein R₆ and R₆' for each occurrence may optionally be substituted with 1 to 3 substituents selected from the group consisting of halogen, --OR₂, alkoxy, heterocycloalkyl, --NR₅C(O)NR₅R₅', --C(O)NR₅R₅', --NR₅C(O)R₅', --CN,-NR₅SO₂R₅', --OC(O)R₅, --SO₂NR₅R₅', --SOR₇, --COOH and --C(O)OR₇, or R₆ and R₆' taken together can be cyclized to form --(CH₂)_{q}X(CH₂)ₛ--;

R₇ for each occurrence is independently selected from the group consisting of C₁ to C₆ alkyl, aryl and heteroaryl, wherein R₇ may optionally be substituted with --(CH₂)_{w}OH;

R₈ is selected from the group consisting of alkoxy, alkoxycarbonyl,-C(O)NR₆R₆', --NR₅R₅', --C(O)R₆, --NR₅C(O)NR₅R₅' and --N-heteroaryl;

R₉ is selected from the group consisting of heterocycloalkyl, heteroaryl, --CN, --(CH₂)ₚN(R₆)C(O)R₆', --(CH₂)ₚCN, --(CH₂)ₚN(R₆)C(O)OR₆',-(CH₂)ₚN(R₆)C(O)NR₆R₆', --(CH₂)ₚ N(R₆)SO₂R₆, --(CH₂)ₚC(O)NR₆R₆',-(CH₂)ₚC(O)OR₆, --(CH₂)ₚOC(O)OR₆, --(CH₂)ₚOC(O)R₆, --(CH₂)ₚOC(O)NR₆R₆',-(CH₂)ₚN(R₆)SO₂NR₆R₆', --(CH₂)ₚOR₆, --(CH₂)ₚOC(O)N(R₆)(CH₂).sub.mOH(CH₂)ₚSOR₆ and --(CH2)ₚOCH₂C(O)N(R.sub- .6)(CH₂)ₘOH;

X is selected from the group consisting of --CR₅R₅'--, --O--, --S--, --S-, --SO₂--, --NC(O)OR₇--, --NC(O)NR₅-- and --NR₅--;

Z is nitrogen; m is an integer between 1 and 6; n is an integer from 1 to 6; p is an integer from 0 to 5; w is an integer between 0 and 5; and q and s are each independently an integer between 1 and 3, with the proviso that R₅, R₅', R₆ or R₆' cannot be hydrogen when either is connected to a carbonyl group (e.g., --C(O)R₆) or sulfone group (e.g., --SO₂R₆).

Treatments

The compounds described herein can be administered to cells in culture, e.g. *in vitro* or *ex vivo,* or to a subject, e.g., *in vivo,* to treat, prevent, and/or diagnose a variety of disorders, including those described herein.

As used herein, the term "treat" or "treatment" is defined as the application or administration of a compound, alone or in combination with, a second compound to a subject, e.g., a patient, or application or administration of the compound to an isolated tissue or cell, e.g., cell line, from a subject, e.g., a patient, who has a disorder (e.g., a disorder as described herein), a symptom of a disorder, or a predisposition toward a disorder, with the purpose to cure, heal, alleviate, relieve, alter, remedy, ameliorate, improve or affect the disorder, one or more symptoms of the disorder or the predisposition toward the disorder (e.g., to prevent at least one symptom of the disorder or to delay onset of at least one symptom of the disorder).

An amount of an compound effective to treat a disorder refers to an amount of the compound which is effective, upon single or multiple dose administration to a subject, in treating a cell, or in curing, alleviating, relieving or improving a subject with a disorder beyond that expected in the absence of such treatment. An effective amount can be estimated, e.g., using an animal model of the disorder in question.

An amount of a compound effective to prevent a disorder, or "a prophylactically effective amount" of the compound refers to an amount effective, upon single- or multiple-dose administration to the subject, in preventing or delaying the occurrence of the onset or recurrence of a disorder or a symptom of the disorder.

In some instances, a compound described herein is used in the treatment or prevention of ileus. Ileus (e.g., paralytic ileus or adynamic ileus) is temporary absence of the normal contractile movements of the intestinal wall. Like an obstruction of the intestines, ileus prevents the passage of intestinal contents. Unlike a mechanical obstruction, though, ileus rarely leads to rupture. Ileus commonly occurs for 24 to 72 hours after abdominal surgery. When symptoms of ileus last for more than about 72 hours, it is referred to as paralytic post-operative ileus. Exemplary causes include an infection or a blood clot inside the abdomen, atherosclerosis that reduces the blood supply to the intestine, or an injury to an intestinal artery or vein. Disorders outside the intestine may cause ileus, such as kidney failure or abnormal levels of blood electrolytes, low potassium or high calcium levels, for example. Other causes of ileus are use of certain drugs (especially opioid analgesics and anticholinergic drugs) and an underactive thyroid gland. The symptoms of ileus are abdominal bloating, nausea, vomiting, esophageal reflux, severe constipation, loss of appetite, and cramps. Exemplary animal models of ileus include: a dog model of postoperative ileus (see, e.g., Furuta et al. (2002) Biol Pharm Bull. 25(8):1063-71) and a rat model of postoperative ileus (see, e.g., De Winter et al. (1998) Eur J Pharmacol. 344(1):71-6). In some embodiments, a compound described herein can be administered in combination with an additional therapeutic agent such as, e.g., a mu opioid receptor antagonist (e.g., alvimopan and methylnaltrexone), a motilin receptor agonist (e.g., erythromycin), and a CIC-2 chloride channel activator (e.g., lubiprostone).

In some instances, a compound described herein is used in the treatment of gastroparesis. Gastroparesis, also called delayed gastric emptying, is a disorder in which the stomach takes too long to empty its contents into the intestine. It often occurs in people with type 1 diabetes or type 2 diabetes. Gastroparesis happens when nerves to the stomach are damaged or stop working. The vagus nerve controls the movement of food through the digestive tract. If the vagus nerve is damaged, the muscles of the stomach and intestines do not work normally, and the movement of food is slowed or stopped. Diabetes can damage the vagus nerve if blood glucose levels remain high over a long period of time. High blood glucose causes chemical changes in nerves and damages the blood vessels that carry oxygen and nutrients to the nerves. Other factors that can contribute to nerve damage include surgery, infection, and various drugs (e.g.,opioids). The symptoms of gastroparesis include nausea, emesis, bloating, loss of appetite, and weight loss. Exemplary animal models of gastroparesis include a diabetic gastroparesis mouse model (see, e.g., James et al. (2004) Am J Physiol Gastrointest Liver Physiol. 287(3):G612-9) and vagotomized animals (see, e.g., Ouyang H (2004) Dig Dis Sci. 49(9):1418-24).

In one embodiment, a compound (e.g., its characteristics or appropriate dose) is evaluated by measuring gut motility in vivo, e.g., to study gastric emptying or gastroparesis. These methods can include one or more a) the transit time of material through the GI tract, b) absorption into blood of material from the gut, and c) the measurement of electrical or contractile activity of the gut.

Transit time studies: In these experiments, animals are fed/gavaged with a material that can be tracked during transit through the GI tract. Examples of such materials include: charcoal, colored food, radioactive material, xylose or acetaminophen. Material is introduced into the GI tract, and then the GI tract is evaluated to determine the location of the material along the GI at various time points. In each case, it is expected that a ghrelin agonist would facilitate movement of material through the gut. Charcoal or Colored food can be used. The location of either charcoal or a dye-coloured food can be determined visually. Animals are fed the material and sacrificed at different time points. The location and quantity of material within the various GI segments is then determined. In another assay, animals are dosed orally with radioactive liquid (eg, methylcellulose containing technetium-99m). A various times following experimental manipulation the animals are sacrificed. The GI tract is removed, subdissected and the various regions assayed for presence of radioactivity. This method provides a quantitative method of determine transit of material through the GI.

Absorption studies: In these studies animals are dosed orally with material and the mean absorbance time (MAT) is determined. Acetaminophen and xylose are not absorbed from the stomach and therefore their presence in blood samples taken at various time points indicates gastric emptying.

Direct measures of myoelectric/contractile activity include electrophysiology and manometry. *Electrophysiology:* Recording electrodes are inserted directly into the smooth muscle wall of the GI tract. Myoelectric potentials indicate contractile activity of the region. Action potentials can also be recorded from nerves subserving the GI tract, similarly indicative of regional activity. *Manometry:* A specialized manometric catheter is attached to the smooth muscle wall of the GI tract and connected to a pressure transducer for recording of contractile activity. A ghrelin agonist would be expected to increase the contractile activity of the GI tract.
In some embodiments, a compound described herein can be administered in combination with an additional therapeutic agent for treating or preventing gastroparesis such as, e.g., a motilin receptor agonist (e.g., erythromycin), and a dopamine antagonist (e.g., metaclopramide).

ln some instances, a compound described herein is used in the treatment of cachexia. Cachexia is a condition of severe malnutrition characterized by anorexia, weight loss and muscle and fat wasting that occurs as a consequence of chronic conditions such as cystic fibrosis, cerebral palsy, cancer, AIDS, congestive heart failure, failure to thrive in older populations, end-stage organ failure, neurological degenerative diseases, chronic obstructive lung disease, chronic liver disease, and chronic renal disease. Over production of cytokines such as TNF-alpha, IL1, IL6 and TGF-gamma have been associated with the pathogenesis of this syndrome. Other factors associated with the pathogenesis of cachexia can include dysfunction of peptidergic circuits and malabsorption of nutrients. Cachexia has repeatedly been associated with adverse clinical outcomes, and increased morbidity and mortality. Some symptoms of cachexia include the appearance of widespread of wasting of the body, pale color, dry wrinkled skin and mental depression, which can be a clinical sign of serious chronic disease, especially cancer. Severe cachexia occurs in most patients with advanced cancer or AIDS. The physiological, metabolic, and behavioral changes in cachexia are associated with patient complaints of weakness, fatigue, gastrointestinal distress, sleep/wake disturbances, pain, listlessness, shortness of breath, lethargy, depression, malaise and the fear of being a burden on family and friends. Although cachexia has been classically associated with chronic infections and malignant conditions, it has also been identified in patients after extensive traumatic injury and sepsis, and in aging persons with failure to thrive syndrome.

In some instances the compounds can be administered with another agent useful in the treatment of cachexia, such as a corticosteroid, a progestational agent (e.g., megace), an appetite stimuluate (e.g. dronabinol) a prokinetic agent (e.g., metoclopramide), a cytokine blocker (e.g., hydrazine sulfate, pentoxifylene, thalidomide, melatonin, eicosapentaoic acid and NSAIDS).

In some instances, a compound described herein is used in the treatment of lipodystrophy. Lipodystrophy is a complicated disorder of adipose (fat) tissue that can include an increase in central fat and atrophy of appendicular fat. There are two main classes of lipodystrophy, inherited lipodystrophies (genetically determined), and acquired lipodystrophies (for example, HIV-associated). Exemplary animal models of lipodystrophy include mouse models (see, e.g., Reue et al. (2000) Curr Atheroscler Rep. 2(5):3 90-6).

Examples of inherited lipodystrophies, which are very rare, occurring, for example, in less than 1 in 10,000 people, include Congenital Generalized Lipodystrophy (CGL), Familial Partial Lipodystrophy Dunnigan variety (FPLD), FPL Mandibuloacral Dysplasia, Kobberling, Multiple Symmetric Lipomatosis (MSL, Madelung's disease), SHORT Syndrome, and Neonatal Progeroid Syndrome (Wiedemann-Rautenstrauch Syndrome).

In general, about 30% to about 50% of HIV patients on highly active antiretroviral therapy (HAART) develop some form of lipodystrophic disorder. HIV-associated lipodystrophy is a disorder that generally includes subcutaneous fat loss in the face and limbs of RIV-positive patients after treatment with a protease inhibitor. In some instances, HIV lipodystrophy includes both fat loss and fat accumulation in various regions of the body, including the thighs/legs, breast, face, abdomen, and back. Other factors observed in patients with HIV-associated lipodystrophy syndrome include increased triglyceride levels, increased LDL and VLDL cholesterol, low HDL cholesterol and insulin resistance. In general, HIV treatment with a protease inhibitor (e.g., CRIXIVAN®, VIRACEPT®, etc) is a risk factor for HIV lipodystrophy. However, it has also been determined that treatment with a nucleoside reverse transcriptase inhibitor, or combination of a nucleoside reverse transcriptase inhibitor and a protease inhibitor, are also risk factors for HIV lipodystrophy. Other risk factors that may contribute to lipodystrophy (e.g., HIV lipodystrophy) include age (e.g. older patients are more likely to develop HIV lipodystrophy), gender (e.g., in some instances women are more likely to develop HIV lipodystrophy than men), race, and dietary practices.

In some instances, the compounds can be used in a combination treatment, e.g., for HIV lipodystrophy or other disorder described herein, with other therapeutic agents such as narcotics, growth hormones, anabolic steroids, and/or insulin sensitizers. Components of the combination can be administered together or separately.

____Exemplary Patients

In some instances, a patient or subject can be identified for whom treatment using a compound that induces the production or release of GH would be beneficial. For example, the endogenous level of ghrelin and/or GH in a subject can be determined and evaluated to determine a course of treatment. If the patient is determined to have lower ghrelin or GH levels than a predetermined standard, for example, the level of endogenous ghrelin or GH in a healthy patient of the same age and sex, then the patient can be identified as a candidate having increased response to a treatment using a compound that induces the production or release of GH. Endogenous levels of a hormone such as ghrelin or GH can be monitored prior to, during, or after a treatment or a course of treatment.

In some instances, an elderly subject, such as a subject at least 55 years of age (e.g., at least 60 years, at least 65 years, at least 70 years, at least 75 years, at least 80 years, at least 85 years, at least 90 years, at least 95 years, or at least 100 years) can, in some instances, have a higher susceptibility to post operative ileus and to cachexia. Accordingly, such a patient can be identified for treatment using a compound that induces the production or release of GH such as a compound depicted in Table 1. In addition to analyzing the age of a subject, the endogenous GH levels can also be evaluated to further confirm whether the subject is one that would benefit from treatment with a compound that induces the production or secretion of GH.

Kits

A compound described herein can be provided in a kit. The kit includes (a) a composition that includes a compound described herein, and, optionally (b) informational material. The informational material can be descriptive, instructional, marketing or other material that relates to the methods described herein and/or the use of the compound described herein for the methods described herein.

The informational material of the kits is not limited in its form. In one embodiment, the informational material can include information about production of the compound, molecular weight of the compound, concentration, date of expiration, batch or production site information, and so forth. In one embodiment, the informational material relates to use of the compound described herein to treat a disorder described herein.

In one embodiment, the informational material can include instructions to administer the compound described herein in a suitable manner to perform the methods described herein, e.g., in a suitable dose, dosage form, or mode of administration (e.g., a dose, dosage form, or mode of administration described herein). Preferred doses, dosage forms, or modes of administration are intranasal and subcutaneous. In another embodiment, the informational material can include instructions to administer a compound to a suitable subject, e.g., a human, e.g., a human having or at risk for a disorder described herein. For example, the material can include instructions to administer the compound described herein to such a subject.

The informational material of the kits is not limited in its form. In many cases, the informational material, e.g., instructions, is provided in printed matter, e.g., a printed text, drawing, and/or photograph, e.g., a label or printed sheet. However, the informational material can also be provided in other formats, such as computer readable material, video recording, or audio recording. In another embodiment, the informational material of the kit is contact information, e.g., a physical address, email address, website, or telephone number, where a user of the kit can obtain substantive information about an compound described herein and/or its use in the methods described herein. Of course, the informational material can also be provided in any combination of formats.

In addition to a compound described or referred to herein, the composition of the kit can include other ingredients, such as a solvent or buffer, a stabilizer, a preservative, and/or a second compound for treating a condition or disorder described herein. Alternatively, the other ingredients can be included in the kit, but in different compositions or containers than the compound described herein. In such embodiments, the kit can include instructions for admixing the compound described herein and the other ingredients, or for using a compound together with the other ingredients.

The compound can be provided in any form, e.g., liquid, dried or lyophilized form. It is preferred that the compound be substantially pure and/or sterile. When the compound is provided in a liquid solution, the liquid solution preferably is an aqueous solution, with a sterile aqueous solution being preferred. When the compound is provided as a dried form, reconstitution generally is by the addition of a suitable solvent. The solvent, e.g., sterile water or buffer, can optionally be provided in the kit.

The kit can include one or more containers for the composition containing the compound. In some embodiments, the kit contains separate containers, dividers or compartments for the composition and informational material. For example, the composition can be contained in a bottle, vial, or syringe, and the informational material can be contained in a plastic sleeve or packet. In other embodiments, the separate elements of the kit are contained within a single, undivided container. For example, the composition is contained in a bottle, vial or syringe that has attached thereto the informational material in the form of a label. In some embodiments, the kit includes a plurality (e.g., a pack) of individual containers, each containing one or more unit dosage forms (e.g., a dosage form described herein) of a compound described herein. For example, the kit includes a plurality of syringes, ampules, foil packets, or blister packs, each containing a single unit dose of a compound described herein. The containers of the kits can be air tight, waterproof (e.g., impermeable to changes in moisture or evaporation), and/or light-tight.

The kit optionally includes a device suitable for administration of the composition, e.g., a syringe, inhalant, pipette, forceps, measured spoon, dropper (e.g., eye dropper), swab (e.g., a cotton swab or wooden swab), or any such delivery device. In a preferred embodiment, the device is an implantable delivery device.

A number of embodiments of the invention have been described. Nevertheless, it will be understood that various modifications may be made without departing from the spirit and scope of the invention. Accordingly, other embodiments are within the scope of the following claims.

Further aspects of the invention are defined in the following clauses:
a. A method of treating a disorder of the stomach, intestine or duodenum in a human subject, the method comprising, administering to the subject, a pharmaceutical composition comprising a compound that induces the secretion or production of growth hormone, modulation of myenteric nerve activity or both.
b. The method of clause a wherein the disorder is ileus.
c. The method of clause b wherein the disorder is post operative ileus
d. The method of clause a wherein the disorder is gastroparesis.
e. The method of any of the preceding clauses, wherein the compound has one or more of the following properties: (a) it agonizes a somatostatin receptor,and (b) it agonizes a ghrelin receptor.
f. A method of treating cachexia in a subject, the method comprising administering, to the subject, a pharmaceutical composition that comprises a compound that induces the secretion or production of growth hormone.
g. A method of treating lipodystrophy in a subject, the method comprising administering, to the subject, a pharmaceutical composition comprising a compound that induces the secretion or production of growth hormone.
h. The method of clause g wherein the lipodystrophy is HIV lipodystrophy.
i. The method of any of clauses f-h, wherein the compound has one or more of the following properties: (a) it agonizes the ghrelin receptor, (b) it agonizes the GH receptor, (c) it binds to the growth hormone releasing hormone (GHRH) receptor, (d) it agonizes the GHRH receptor, and (e) it antagonizes somatostatin.
j. The method of any of clauses f-h, wherein the compound increases or induces growth hormone release.
k. The method of any preceding clause wherein the compound is ghrelin.
l.. The method of any of clauses a-h wherein the compound is compound 1.
m. The method of any of clauses a-h wherein the compound is compound 2.
n. The method of any of clauses a-h wherein the compound is compound 3.
o. The method of any of clauses a-h wherein the compound is compound 4.
p. The method of any of clauses a-h wherein the compound is compound 5.
q. The method of any of clauses a-h wherein the compound is compound 6.
r. The method of any of clauses a-h wherein the compound is compound 7.
s. The method of any of clauses a-h wherein the compound is compound 8, 9, 10, 11, or 12.
t. The method of any of clauses a-e, further comprising administering to the subject a second compound.
u. The method of clause t, wherein the second compound is a mu opioid antagonist, a ghrelin agonist or a motilin receptor agonist.
v. The method of clause u, wherein the second compound is a mu opioid antagonist and the mu opioid antagonist is alvimopan.
w. The method of clause u, wherein the second compound is a motilin receptor agonist and the motilin receptor agonist is erythromycin.
x. The method of clause f, further comprising administering to the subject a second compound.
y. The method of clause x, wherein the second compound can be one or more of a glucocorticoid, a progestational agent, dronabinol and cyproheptadine.
z. The method of clause y, wherein the second compound is a progestational agent and the progestational agent is megace.
aa. The method of any of clauses a-h, further comprising administering to the subject a second compound.
ab. The method of clause aa, wherein the second compound is GHRP-6, GHRP-I, B-HT920, GHRP-2, growth hormone releasing hormone (GHRH, also designated GRF), IGF-1, or IGF-2.
ac. The method of clause aa, wherein the second compound is clonidine, sumitriptan, physostigmine or pyridostigmine.

## Claims

1. A compound for use in a method of treating a disorder of the stomach, intestine or duodenum in a human subject, the method comprising administering to the subject, a pharmaceutical composition comprising a compound that induces the secretion or production of growth hormone, modulation of myenteric nerve activity or both, wherein the compound is compound 7.

2. The compound of claim 1, wherein the disorder is ileus, preferably wherein the disorder is post operative ileus.

3. The compound of claim 1, wherein the disorder is gastroparesis.

4. The compound of claim 1, wherein the disorder is cachexia.

5. The compound of claim 1, wherein the disorder is lipodystrophy, preferably wherein the lipodystrophy is HIV lipodystrophy.

6. The compound of any of claims 1-3, wherein the method further comprises administering to the subject a second compound.

7. The compound of claim 6, wherein the second compound is a mu opioid antagonist, a ghrelin antagonist or a motilin receptor agonist.

8. The compound of claim 7, wherein the second compound is a mu opioid antagonist and the mu opioid antagonist is alvimopan.

9. The compound of claim 7, wherein the second compound is a motilin receptor agonist and the motilin receptor agonist is arythromycin.

10. The compound of claim 5, wherein the method further comprises administering to the subject a second compound.

11. The compound of claim 10, wherein the second compound can be one or more of a glucocorticoid, a progestational agent, dronabinol and cyproheptadine.

12. The method of claim 11, wherein the second compound is a progestational agent and the progestational agent is megace.

13. The compound of claim 5 wherein the method comprises administering to the subject a second compound.

14. The compound of claim 13, wherein the second compound is GHRP-6, GHRP-I, B-HT920, GHRP-2, growth hormone releasing hormone (GHRH, also designated GRF), IGF-1 or IGF-2.

15. The compound of claim 13, wherein the second compound is clonidine, sumitriptan, physostigmine or pyridostigmine.
